# EUROPÄISCHE PATENTSCHRIFT

(11) **EP 2 699 571 B1**
(45) Veröffentlichungstag und Bekanntmachung des Hinweises auf die Patenterteilung: **05.09.2018**
(21) Anmeldenummer: 12711346.2
(22) Anmeldetag: 26.03.2012
(51) Int. Cl.: C07D 471/04, C07D 471/16, C07D 491/16, C07D 491/22, C07D 495/04, C07D 495/16, C07D 495/22, C07D 498/16, C07D 519/00, C07F 5/02, C09K 11/06, H01L 51/50, H01L 51/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 18.04.2011 EP 11003232
(43) Veröffentlichungstag der Anmeldung: 26.02.2014
(73) Patentinhaber: Merck Patent GmbH, 64293 Darmstadt (DE)
(72) Erfinder: MARTYNOVA, Irina, 64347 Griesheim (DE); PFLUMM, Christof, 64291 Darmstadt (DE); PARHAM, Amir Hossain, 60486 Frankfurt am Main (DE); ANÉMIAN, Rémi Manouk, Seoul 657-169 (KR); MUJICA-FERNAUD, Teresa, 64283 Darmstadt (DE); DE NONANCOURT, Claire, 55300 Loupmont (FR)
(86) Internationale Anmeldenummer: PCT/EP2012/001320
(87) Internationale Veröffentlichungsnummer: WO 2012/143080

(56) Entgegenhaltungen:
- WO-A1-2006/033563
- WO-A1-2010/083871
- WO-A2-2007/031165
- WO-A2-2011/042107
- JP-A- 2003 022 893
- US-A1- 2010 051 928
- DATABASE CAPLUS [Online] CHEMICAL ABSTRACTS SERVICE, COLUMBUS, OHIO, US; XP002680917, gefunden im STN Database accession no. 141:267571 (DN) -& RUI-HUA XIE ET AL: "Tuning spectral properties of fullerenes by substitutional doping", PHYSICAL REVIEW, B. CONDENSED MATTER, AMERICAN INSTITUTE OF PHYSICS. NEW YORK, US, Bd. 69, Nr. 20, 20. Mai 2004 (2004-05-20), Seiten 201403-1-201403-4, XP002636359, ISSN: 0163-1829, DOI: 10.1103/PHYSREVB.69.201403
- DATABASE CAPLUS [Online] CHEMICAL ABSTRACTS SERVICE, COLUMBUS, OHIO, US; XP002680918, gefunden im STN Database accession no. 126:317355 (DN) & RAHIMIZADEH, M. ET AL.: INDIAN JOURNAL OF HETEROCYCLIC CHEMISTRY, Bd. 6, Nr. 3, 1997, Seiten 223-224,
- DATABASE CAPLUS [Online] CHEMICAL ABSTRACTS SERVICE, COLUMBUS, OHIO, US; XP002680919, gefunden im STN Database accession no. 130:338084 (DN) & RAHIMIZADEH, M. ET AL.: JOURNAL OF SCIENCES, ISLAMIC REPUBLIC OF IRAN, Bd. 9, Nr. 2, 1998, Seiten 163-165,
- DATABASE CAPLUS [Online] CHEMICAL ABSTRACTS SERVICE, COLUMBUS, OHIO, US; XP002680920, gefunden im STN Database accession no. 135:38787 (DN) -& JP 2001 160488 A (KONISHIROKU PHOTO IND) 12. Juni 2001 (2001-06-12)

## Beschreibung

Die vorliegende Erfindung betrifft Materialien für die Verwendung in elektronischen Vorrichtungen, insbesondere in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Dies gilt insbesondere für OLEDs, welche im kürzerwelligen Bereich emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz, die Lebensdauer und die Betriebsspannung der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich auch für grün und gegebenenfalls auch für blau phosphoreszierende OLEDs eignen, sowie neue Lochtransportmaterialien und Elektronentransportmaterialien bereitzustellen.

Überraschend wurde gefunden, dass bestimmte, unten näher beschriebene Verbindungen diese Aufgabe lösen und zu Verbesserungen der organischen Elektrolumineszenzvorrichtung führen, insbesondere hinsichtlich der Lebensdauer, der Effizienz und der Betriebsspannung. Dies gilt insbesondere für grün und blau phosphoreszierende Elektrolumineszenzvorrichtungen, vor allem bei Einsatz der erfindungsgemäßen Verbindungen als Matrixmaterial, aber auch für den Einsatz der Verbindungen als Lochtransportmaterial, Lochinjektionsmaterial, Elektronentransportmaterial oder Lochblockiermaterial, je nach genauer Substitution der Verbindung. Diese Materialien sowie organische Elektrolumineszenzvorrichtungen, welche derartige Verbindungen enthalten, sind daher der Gegenstand der vorliegenden Erfindung.

Aus der WO 2007/031165 sind verbrückte Triphenylaminstrukturen mit ähnlicher Grundstruktur wie die erfindungsgemäßen Verbindungen bekannt. Verbindungen, die statt der Phenylgruppen Fünfringheteroarylgruppen enthalten, sind dort jedoch nicht offenbart. Weiterhin werden diese Verbindungen nur als Emitter oder als Lochtransportmaterial beschrieben, nicht jedoch als Matrixmaterial für phosphoreszierende Emitter oder als Elektronentransportmaterial.

Aus der WO 2010/050778 sind verbrückte Triphenylamin- und Phenylcarbazolstrukturen mit ähnlicher Grundstruktur wie die erfindungsgemäßen Verbindungen bekannt. Verbindungen, die statt der Phenylgruppen verbrückte Fünfringheteroarylgruppen enthalten, sind dort jedoch nicht offenbart.
Aus der nicht offen gelegten DE 102009053836.4 sind verbrückte Triarylaminstrukturen mit ähnlicher Grundstruktur wie die erfindungsgemäßen Verbindungen bekannt, wobei mindestens eine der aromatischen Gruppen, die an den Stickstoff gebunden sind, eine Sechsringheteroarylgruppe darstellt. Verbindungen, die statt der Sechsringheteroarylgruppen verbrückte Fünfringheteroarylgruppen enthalten, sind dort jedoch nicht offenbart.

Aus der WO 2010/083871, WO 2011/042107 und WO 2006/033563 sind weitere verbrückte Triphenylaminstrukturen bekannt. Verbindungen, die statt der Phenylgruppen Fünfringheteroarylgruppen enthalten, sind dort jedoch nicht offenbart. Es hat sich überraschend gezeigt, dass gerade der Einsatz der erfindungsgemäßen Verbindungen in organischen Elektrolumineszenzvorrichtungen zu guten elektronischen Eigenschaften führt.
Gegenstand der vorliegenden Erfindung ist daher eine Verbindung ausgewählt aus den Verbindungen der Formeln (5a) bis (11a), 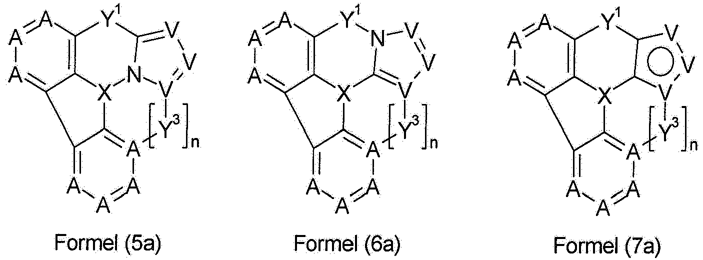 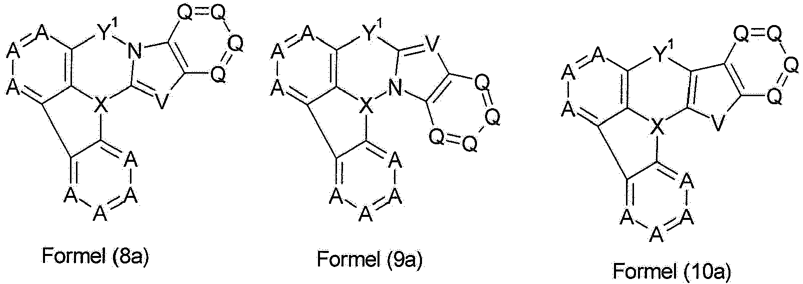 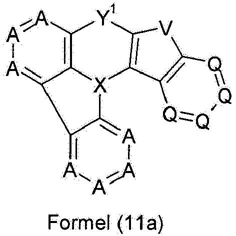 wobei für die verwendeten Symbole und Indizes gilt:
- A: ist bei jedem Auftreten gleich CR;
- X: ist N;
- Y¹: ist C(R¹)₂;
- V: ist bei jedem Auftreten gleich oder verschieden CR, N, NR, S oder O, mit der Maßgabe, dass genau ein Symbol V für NR, S oder O steht;
- Q: ist bei jedem Auftreten gleich oder verschieden CR oder N;
- R: ist bei jedem Auftreten gleich oder verschieden ausgewählt aus der Gruppe bestehend aus H, D, F, CN, N(Ar)₂, C(=O)Ar, P(=O)(Ar)₂, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O oder S ersetzt sein können und wobei ein oder mehrere H-Atome durch D oder F ersetzt sein können, einem aromatischen oder heteroaromatischen Ringsystem mit 6 bis 30 aromatischen Ringatomen, das jeweils mit einem oder mehreren Resten R² substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R² substituiert sein kann;
- R¹: ist ausgewählt aus der Gruppe bestehend aus H, Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R² substituiert sein können;
- R²: ist bei jedem Auftreten gleich oder verschieden ausgewählt aus der Gruppe bestehend aus H, D, F, Cl, Br, I, CN, NO₂, N(Ar)₂, N(R³)₂, C(=O)Ar, C(=O)R³, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder eine Aralkyl- oder Heteroaralkylgruppe mit 5 bis 60 aromatischen Ringatomen;
- Ar: ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar, welche an dasselbe N-Atom oder P-Atom binden, auch durch eine Einfachbindung oder eine Brücke, ausgewählt aus N(R³), C(R³)₂, O oder S, miteinander verbrückt sein;
- R³: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können;
- n: ist gleich 0.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer bevorzugten Ausführungsform der Erfindung stehen pro Cyclus maximal zwei Gruppen Q für N, besonders bevorzugt maximal eine Gruppe Q. Ganz besonders bevorzugt stehen alle Gruppen Q für CR.

Ganz besonders bevorzugte Ausführungsformen der Erfindung sind daher die folgenden Verbindungen (5b) bis (11b), 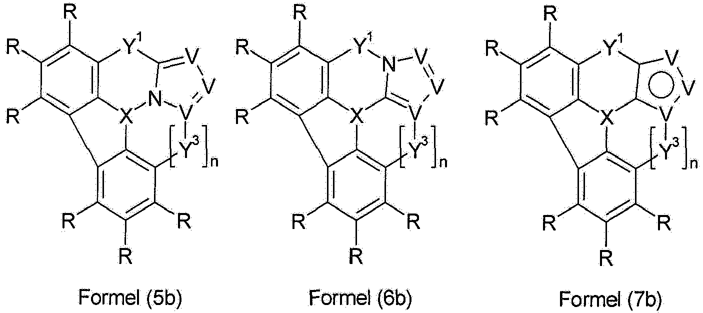 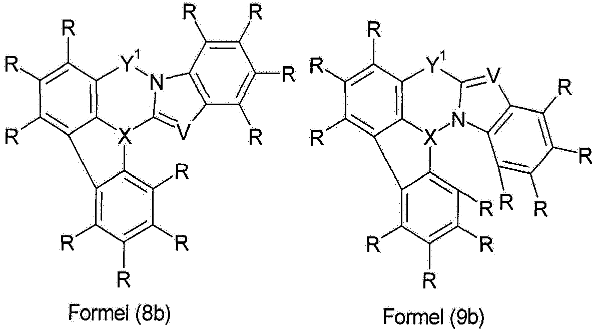 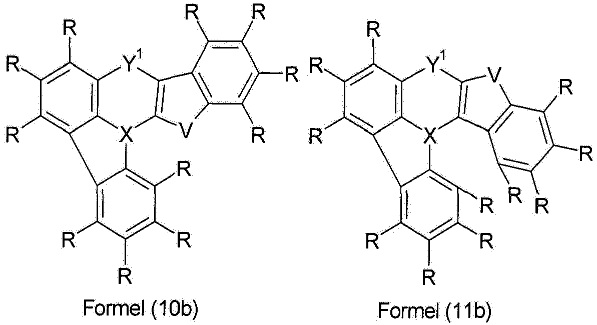 wobei die verwendeten Symbole und Indizes die oben genannten Bedeutungen aufweisen.

Wenn die Verbindungen gemäß Formel (5b) bis (11b) mit Resten R ungleich Wasserstoff oder Deuterium substituiert sind, dann sind diese Reste R bevorzugt jeweils an den Cyclen, die Ar² und Ar³ entsprechen, in der para-Position zur Gruppe X gebunden. Bevorzugt sind daher die Verbindungen gemäß den folgenden Formeln (5d) bis (11d), 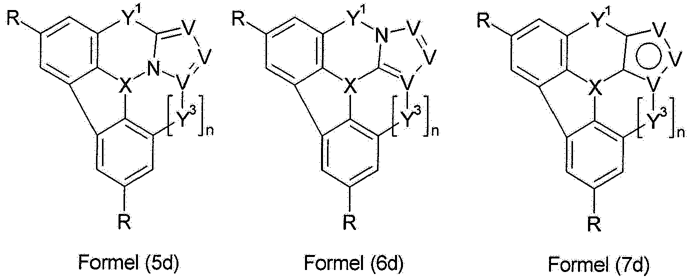 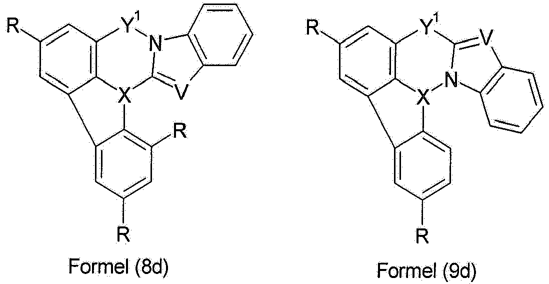 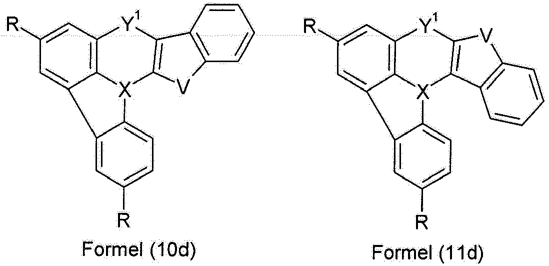 wobei die verwendeten Symbole und Indizes die oben genannten Bedeutungen aufweisen.

Bevorzugte Strukturen des Fünfring-Heterocyclus in den Formeln (5a), (5b), (5c) und (5d) sind die folgenden Strukturen der Formeln (Ar¹-1) bis (Ar¹-6),

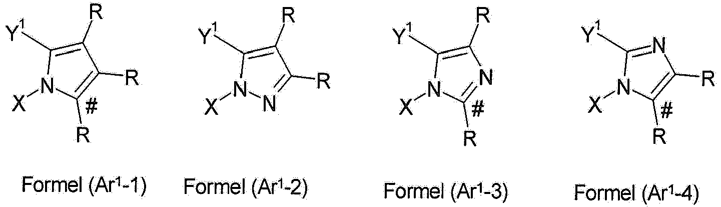

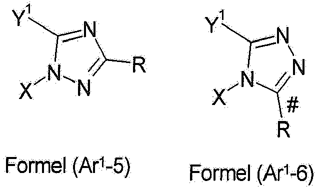

Dabei ist jeweils auch die Bindung dieses Cyclus an X und an Y¹ eingezeichnet. Die mit # gekennzeichnete Position deutet die Position einer möglichen Bindung an Y³ an, wobei in diesem Fall an dem Kohlenstoffatom kein Rest R gebunden ist.

Bevorzugte Strukturen des Fünfring-Heterocyclus in den Formeln (6a), (6b), (6c) und (6d) sind die folgenden Strukturen der Formeln (Ar¹-7) bis (Ar¹-12),

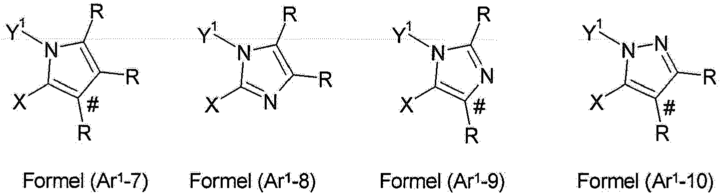

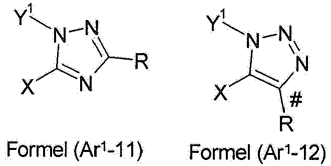

Dabei ist jeweils auch die Bindung dieses Cyclus an X und an Y¹ eingezeichnet. Die mit # gekennzeichnete Position deutet die Position einer möglichen Bindung an Y³ an, wobei in diesem Fall an dem Kohlenstoffatom kein Rest R gebunden ist.

Bevorzugte Strukturen des Fünfring-Heterocyclus in den Formeln (7a), (7b), (7c) und (7d) sind die folgenden Strukturen der Formeln (Ar¹-13) bis (Ar¹-27),

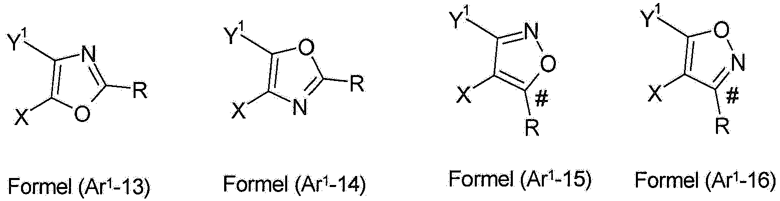

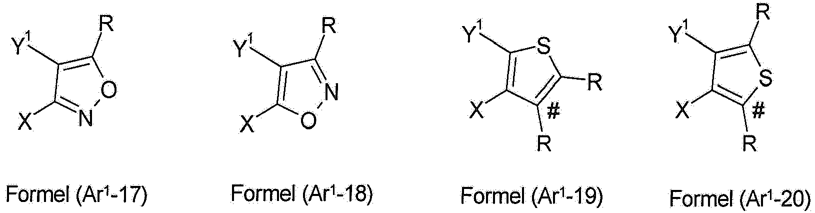

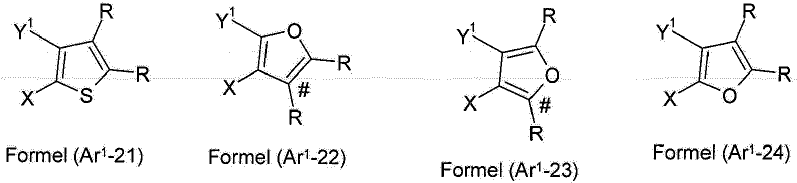

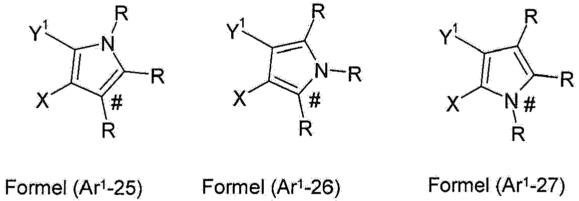

Dabei ist jeweils auch die Bindung dieses Cyclus an X und an Y¹ eingezeichnet. Die mit # gekennzeichnete Position deutet die Position einer möglichen Bindung an Y³ an, wobei in diesem Fall an dem Kohlenstoffatom kein Rest R gebunden ist.

In einer besonders bevorzugten Ausführungsform der Verbindung gemäß Formel (5a) bis (11d) ist R bei jedem Auftreten gleich oder verschieden ausgewählt aus der Gruppe bestehend aus H, D, N(Ar)₂, einer geradkettigen Alkylgruppe mit 1 bis 4 C-Atomen oder einer verzweigten Alkylgruppe mit 3 oder 4 C-Atomen, die jeweils mit einem oder mehreren Resten R¹ substituiert sein kann, wobei ein oder mehrere H-Atome durch D ersetzt sein können, oder einem aromatischen oder heteroaromatischen Ringsystem mit 6 bis 18 aromatischen Ringatomen, das jeweils mit einem oder mehreren Resten R² substituiert sein kann.

Substituenten R¹, die in Y¹, Y² und/oder Y³ gebunden sind, sind bevorzugt ausgewählt aus der Gruppe bestehend aus H, Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R² substituiert sein können. Dabei können zwei Reste R¹, welche in derselben Gruppe Y¹, Y² oder Y³ gebunden sind, auch miteinander ein Ringsystem bilden und so ein Spirosystem aufspannen. Wenn Y¹, Y² oder Y³ für eine Gruppe N(R¹) steht, so steht R¹ besonders bevorzugt für eine aromatisches oder heteroaromatisches Ringsystem, welches durch einen oder mehrere Reste R² substituiert sein kann. Wenn Y¹, Y² oder Y³ für eine Gruppe C(R¹)₂ steht, so steht R¹ besonders bevorzugt gleich oder verschieden bei jedem Auftreten für H, eine Alkylgruppe mit 1 bis 10 C-Atomen oder ein aromatisches oder heteroaromatisches Ringsystem mit 5 bis 20 aromatischen Ringatomen, welches jeweils durch einen oder mehrere Reste R² substituiert sein kann. Dabei können zwei Reste R¹, welche an dasselbe Kohlenstoffatom binden, auch miteinander ein Ringsystem bilden und so ein aromatisches oder aliphatisches Spirosystem aufspannen.

Dabei haben für Verbindungen, die durch Vakuumverdampfung verarbeitet werden, die Alkylgruppen in den Resten R bzw. R¹ bevorzugt nicht mehr als vier C-Atome, besonders bevorzugt nicht mehr als ein C-Atom. Für Verbindungen, die aus Lösung verarbeitet werden, eignen sich insbesondere auch Verbindungen, die mit Alkylgruppen mit bis zu 10 C-Atomen substituiert sind oder die mit Oligoarylengruppen, beispielsweise ortho-, meta-, para- oder verzweigten Terphenylgruppen bzw. Quaterphenylgruppen oder ortho-, meta- oder para-Biphenylgruppen, substituiert sind.

Wenn die erfindungsgemäße Verbindung als Matrixmaterial für einen phosphoreszierenden Emitter oder als Elektronentransportmaterial oder als Lochblockiermaterial eingesetzt wird, ist bevorzugt mindestens ein Substituent R, R¹ und/oder R², bevorzugt R, ist eine elektronenarme Gruppe, insbesondere ausgewählt aus Strukturen gemäß den folgenden Formeln (12) bis (15), 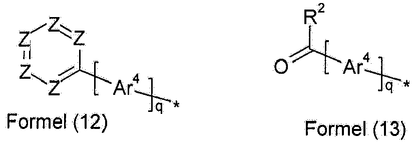 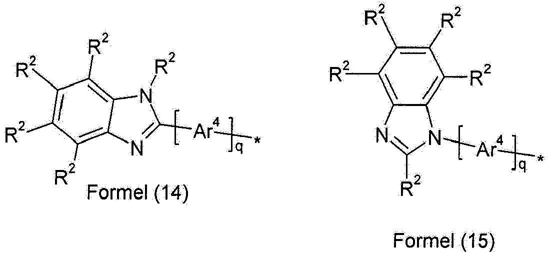 wobei R² die oben genannte Bedeutung hat, * die Position der Bindung der Gruppe gemäß Formel (12) bis (18) andeutet und weiterhin gilt:
- Z: ist bei jedem Auftreten gleich oder verschieden CR² oder N, mit der Maßgabe, dass eine Gruppe Z, zwei Gruppen Z oder drei Gruppen Z für N stehen;
- Ar⁴: ist gleich oder verschieden bei jedem Auftreten eine bivalente Aryl- oder Heteroarylgruppe mit 5 bis 18 C-Atomen, welche durch einen oder mehrere Reste R² substituiert sein kann;
- q: ist bei jedem Auftreten gleich oder verschieden 0, 1, 2 oder 3.

Dabei wird unter einem elektronenarmen Heteroaromaten ein Fünfringheteroaromat mit mindestens zwei Heteroatomen oder ein Sechsringheteroaromat mit mindestens einem Heteroatom verstanden.

In einer besonders bevorzugten Ausführungsform der Erfindung steht mindestens ein Substituent R für eine Gruppe der oben genannten Formel (12), wobei jeweils zwei oder drei Symbole Z für N stehen und die anderen Symbole Z für CR² stehen. Besonders bevorzugte Gruppen R sind daher die Gruppen der folgenden Formeln (19) bis (25), 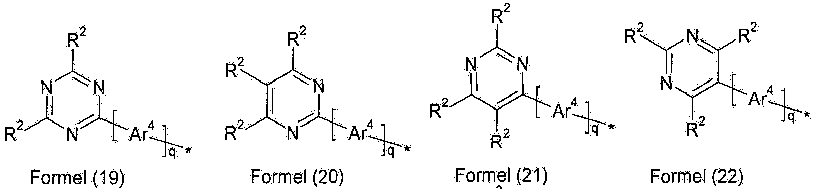 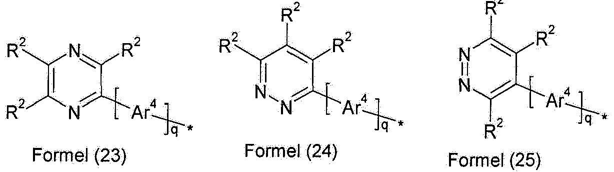 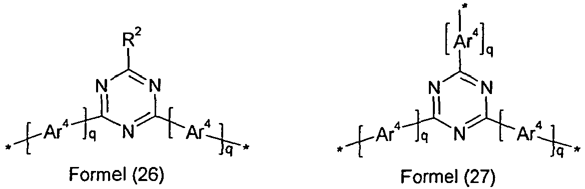 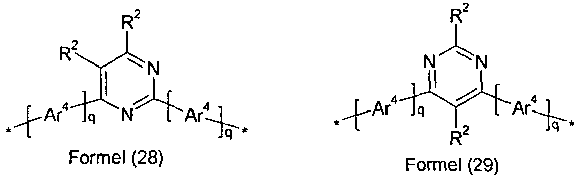 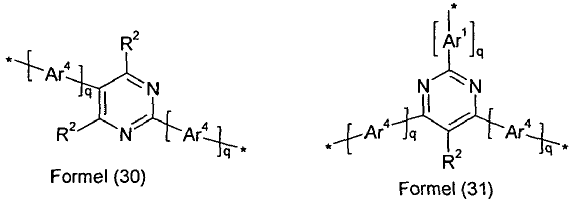 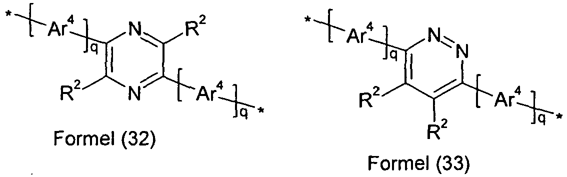 wobei die verwendeten Symbole und Indizes die oben genannten Bedeutungen aufweisen.

Wenn R für eine Gruppe der Formel (19) steht, dann steht R² in dieser Gruppe bevorzugt für ein aromatisches oder heteroaromatisches Ringsystem mit 5 bis 24 aromatischen Ringatomen, welches durch einen oder mehrere Reste R³ substituiert sein kann, insbesondere für Phenyl, ortho-, meta- oder para-Biphenyl, ortho-, meta-, para- oder verzweigtes Terphenyl oder ortho-, meta-, para- oder verzweigtes Quaterphenyl.

Wenn R für eine Gruppe der Formel (20) bis (33) steht, dann steht R² in diesen Gruppen bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R³ substituiert sein kann, insbesondere für Phenyl, ortho-, meta- oder para-Biphenyl, ortho-, meta-, para- oder verzweigtes Terphenyl oder ortho-, meta-, para- oder verzweigtes Quaterphenyl.

Wenn die erfindungsgemäße Verbindung als Matrixmaterial für einen phosphoreszierenden Emitter oder als Lochtransportmaterial eingesetzt wird, ist mindestens ein Substituent R oder R¹, bevorzugt R, bevorzugt ausgewählt aus der Gruppe bestehend aus -NAr₂, Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R² substituiert sein können. Diese Gruppen sind bevorzugt ausgewählt aus den Gruppen der folgenden Formeln (34) bis (47), 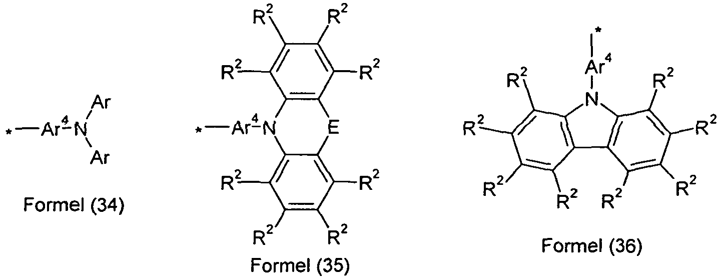 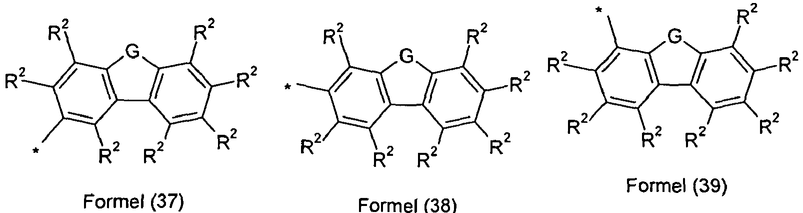 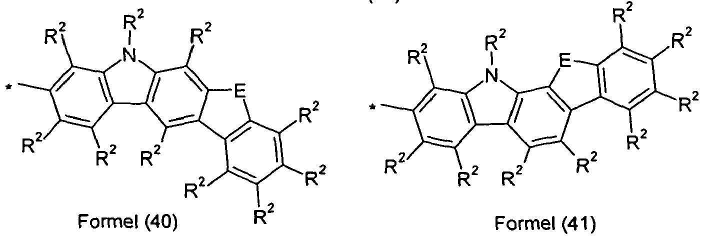 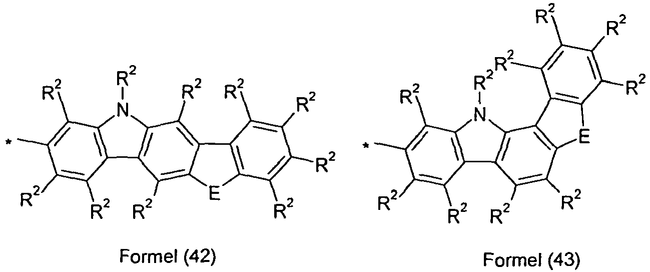 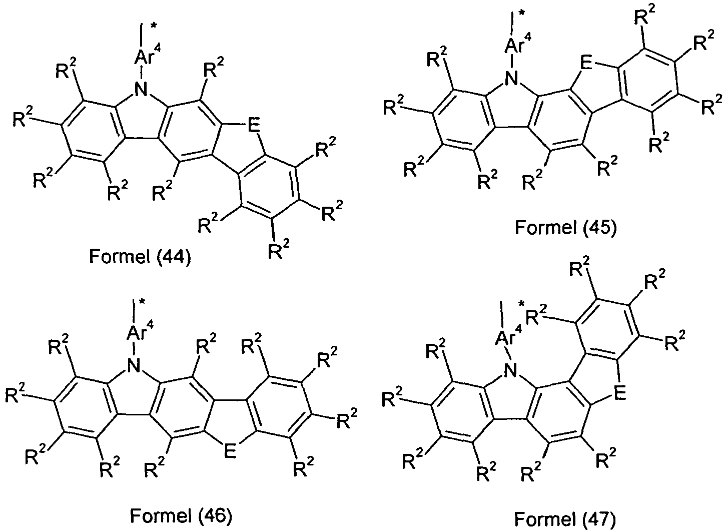 wobei die verwendeten Symbole die oben genannten Bedeutungen aufweisen und weiterhin gilt:
- E: ist ausgewählt aus der Gruppe bestehend aus C(R²)₂, NR², O oder S;
- G: ist ausgewählt aus der Gruppe bestehend aus NR², O oder S.

In einer weiteren bevorzugten Ausführungsform der Erfindung stehen in den erfindungsgemäßen Verbindungen die Symbole R, die nicht für eine Gruppe der oben aufgeführten Formeln (12) bis (47) stehen, für H oder D.

Bevorzugt sind weiterhin auch Verbindungen, die gleichzeitig sowohl elektronentransportierende Substituenten R bzw. R¹ aufweisen, die aus den oben genannten Formeln (12) bis (33) ausgewählt sind, als auch lochtransportierende Substituenten R bzw. R¹, die aus den oben genannten Formeln (34) bis (47) ausgewählt sind.

In einer weiteren bevorzugten Ausführungsform der Erfindung ist der Index p = 2 oder 3, besonders bevorzugt 2.

In einer weiteren bevorzugten Ausführungsform der Erfindung ist der Index n = 0.

In einer weiteren bevorzugten Ausführungsform stehen ein oder zwei Gruppen R oder R¹, bevorzugt R, für eine Gruppe der oben genannten Formeln (12) bis (47), besonders bevorzugt genau eine Gruppe R, und die anderen Gruppen R stehen für H oder D.

Die oben genannten Ausführungsformen der Erfindung sind beliebig miteinander kombinierbar. Insbesondere sind die oben aufgeführten allgemeinen Formeln (1) bis (11) bzw. (5a) bis (11d) beliebig mit den Formeln (Ar¹-1) bis (Ar¹-27) sowie mit den oben genannten bevorzugten Ausführungsformen für X, Y¹, Y², Y³, R, R¹, R², m und n kombinierbar. In einer bevorzugten Ausführungsform der Erfindung treten die oben genannten Bevorzugungen gleichzeitig auf.

Entsprechendes gilt für die Verbindungen der Formel (2). Besonders bevorzugte Ausführungsformen der Verbindungen der Formel (2) sind entsprechend Verbindungen gemäß den oben genannten Formeln (5a) bis (11d), bei denen jeweils zwei oder mehr dieser Einheiten durch eine bivalente Gruppe L, die jeweils statt des Substituenten R in para-Position zu X gebunden ist, miteinander verbrückt sind, wobei weiterhin die oben genannten Bevorzugungen für für X, Y¹, Y², Y³, R, R¹, R², L, m, n und p gelten.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die folgenden Verbindungen.

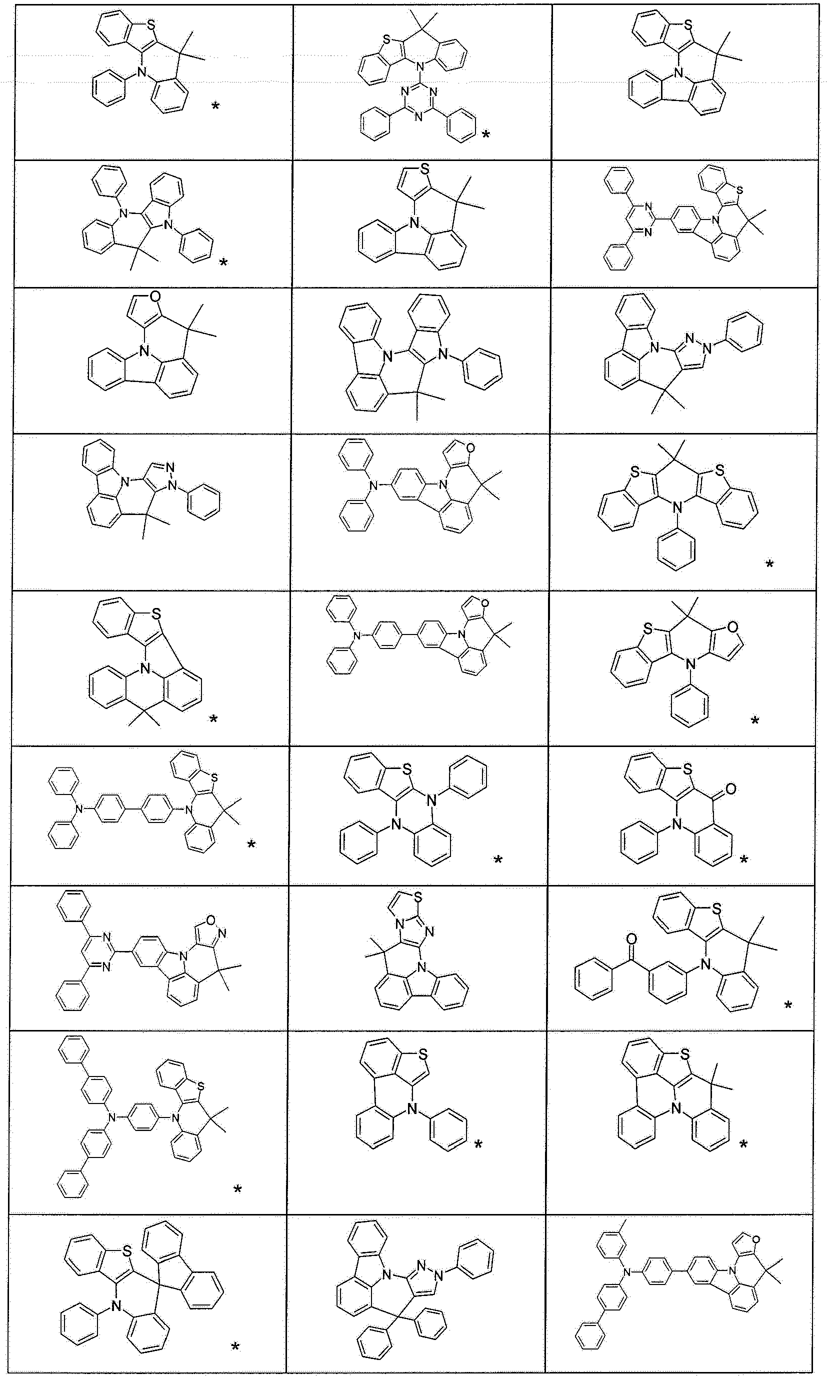

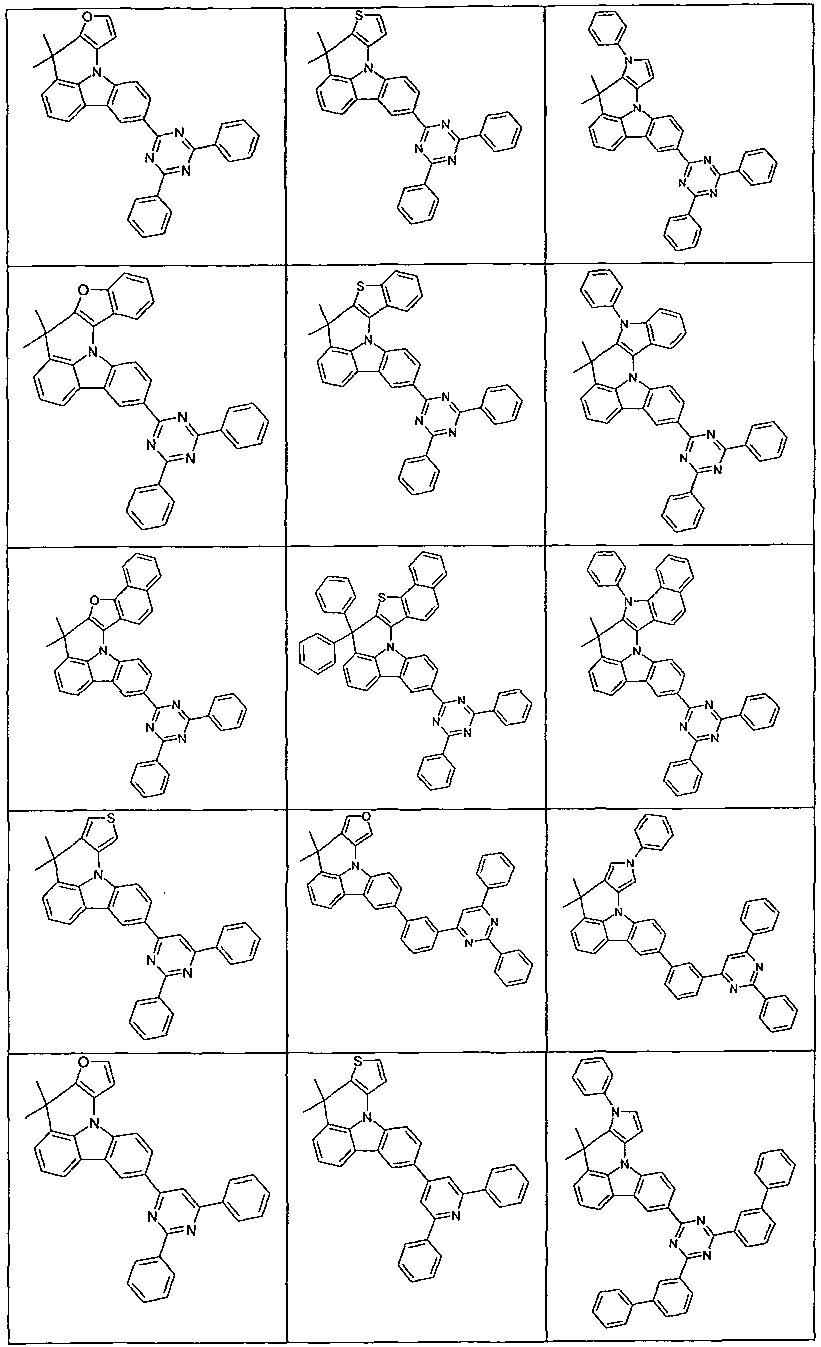

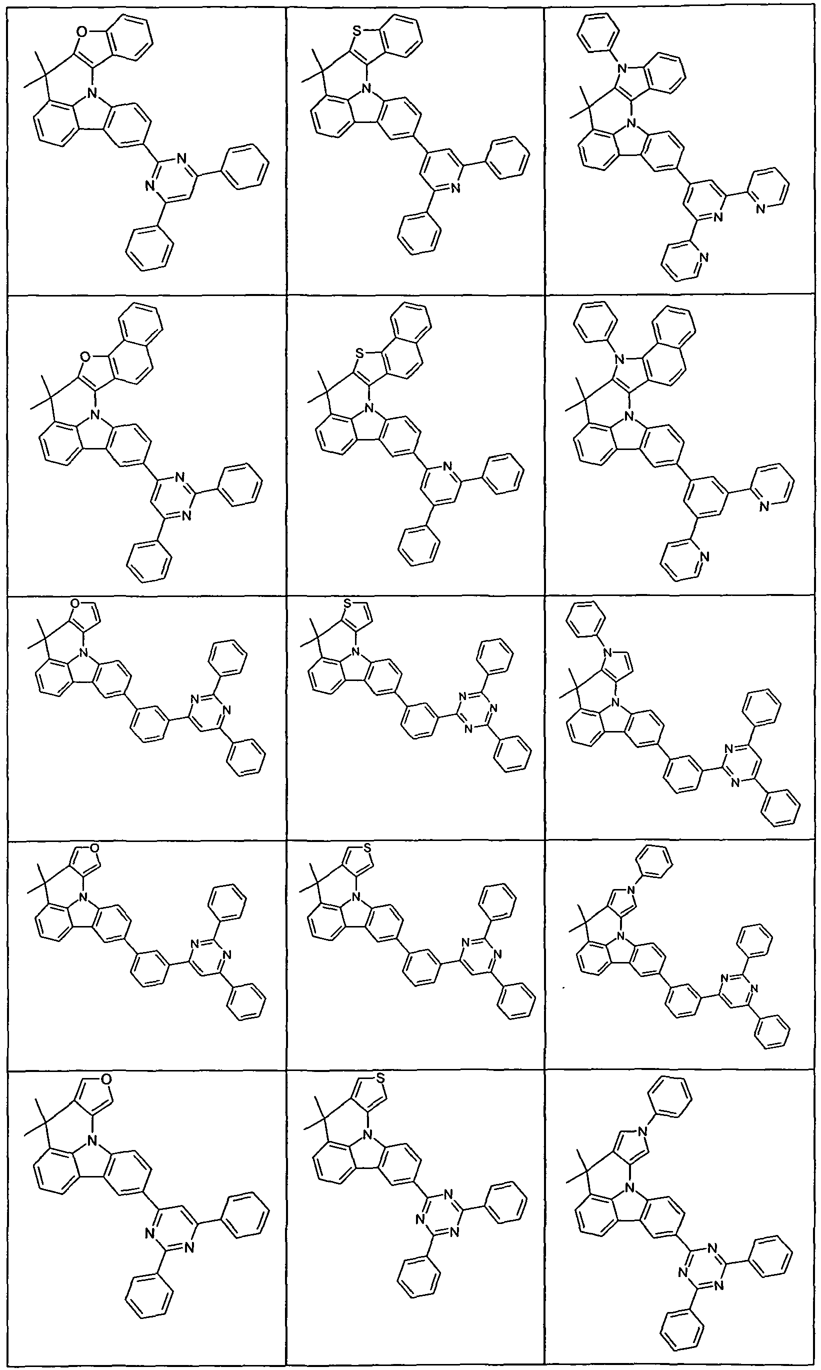

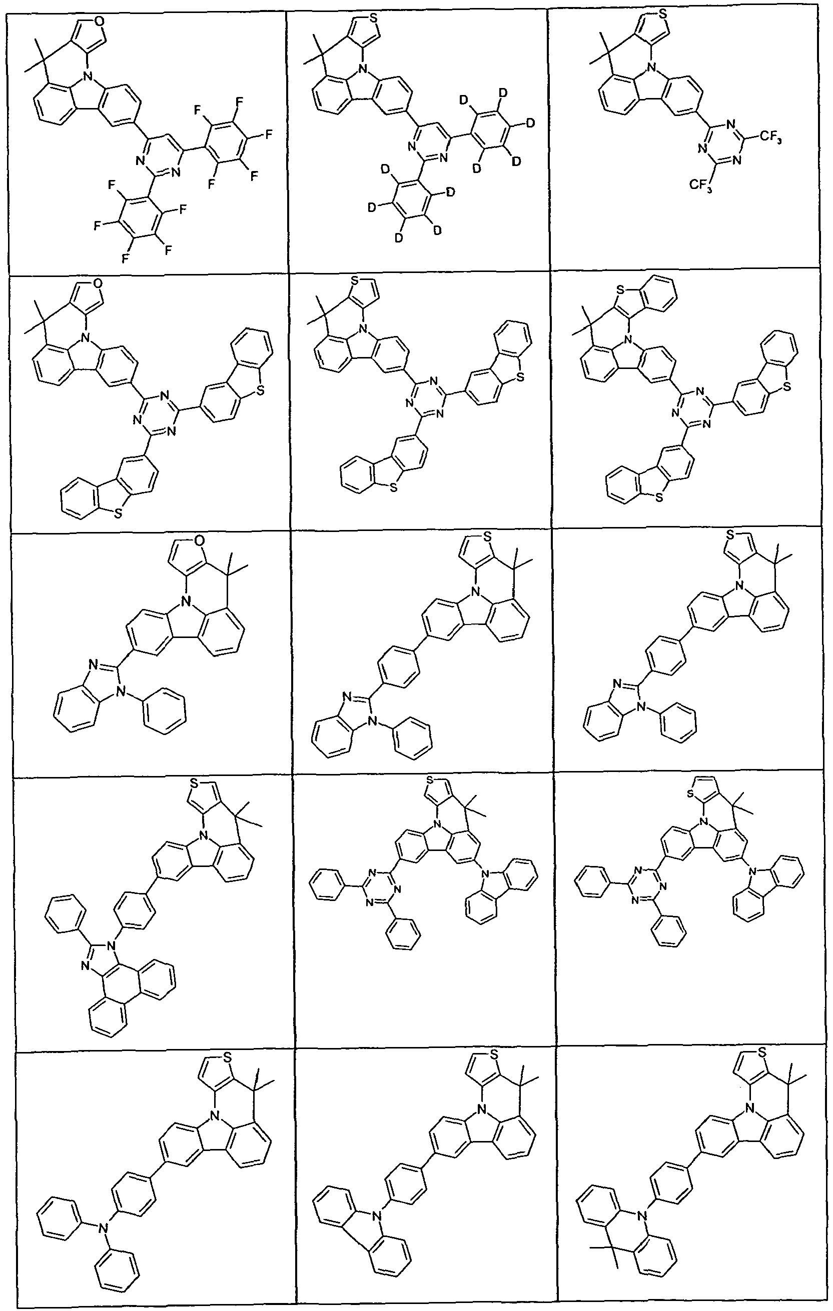

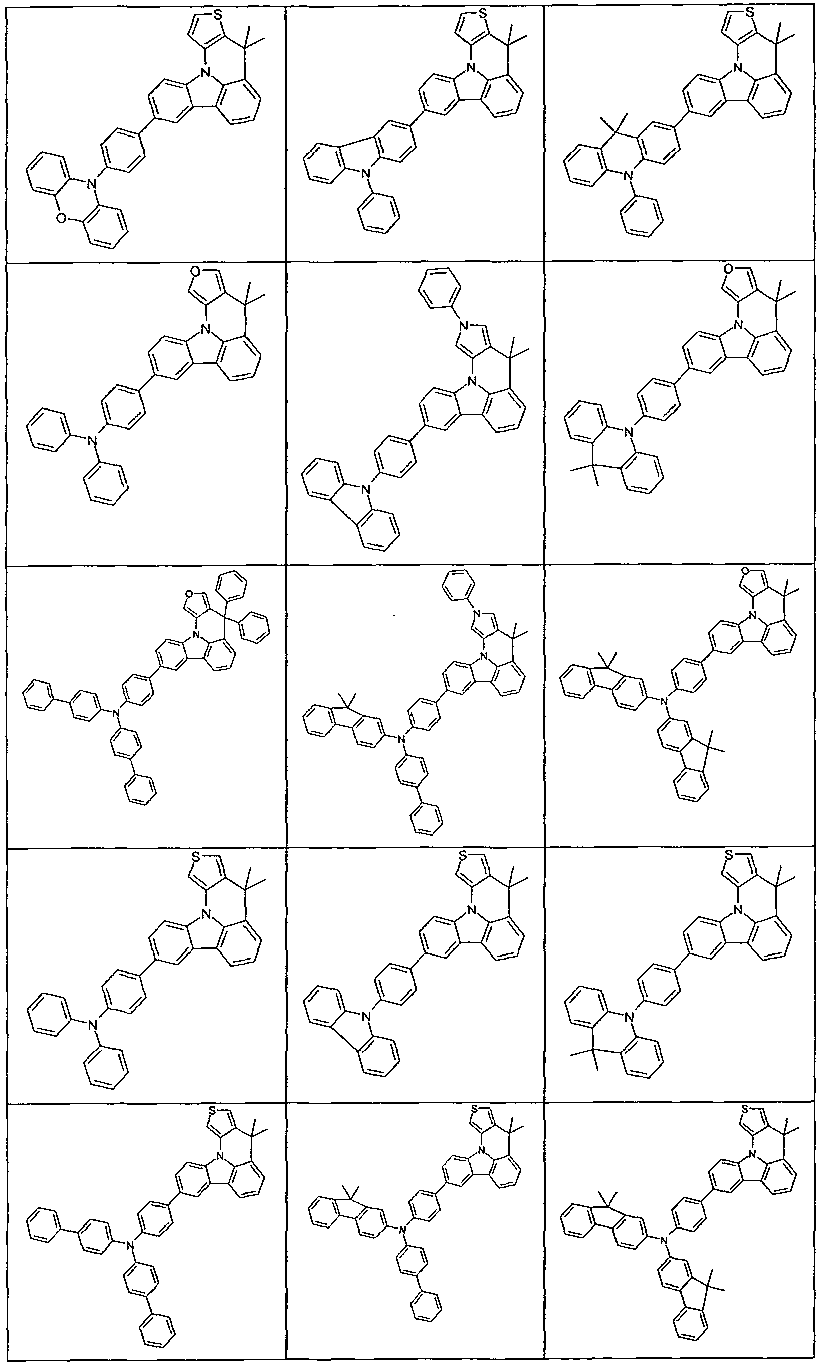

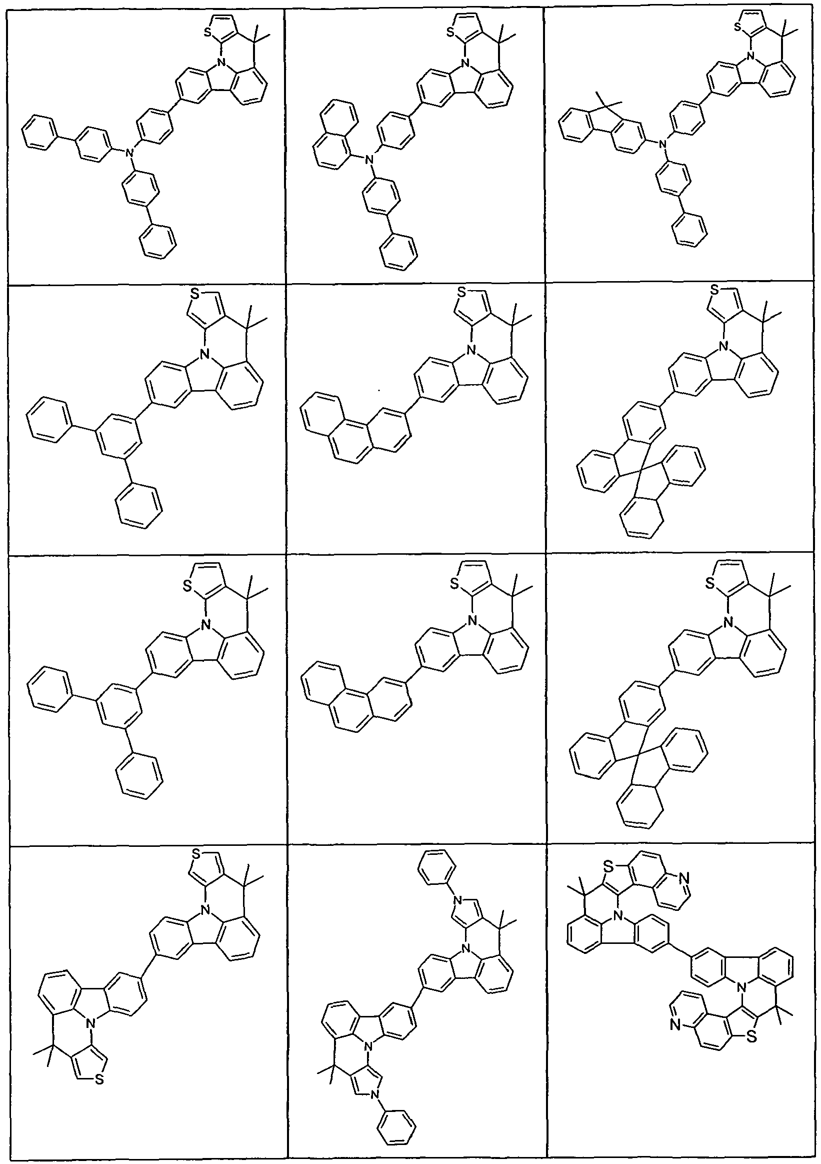

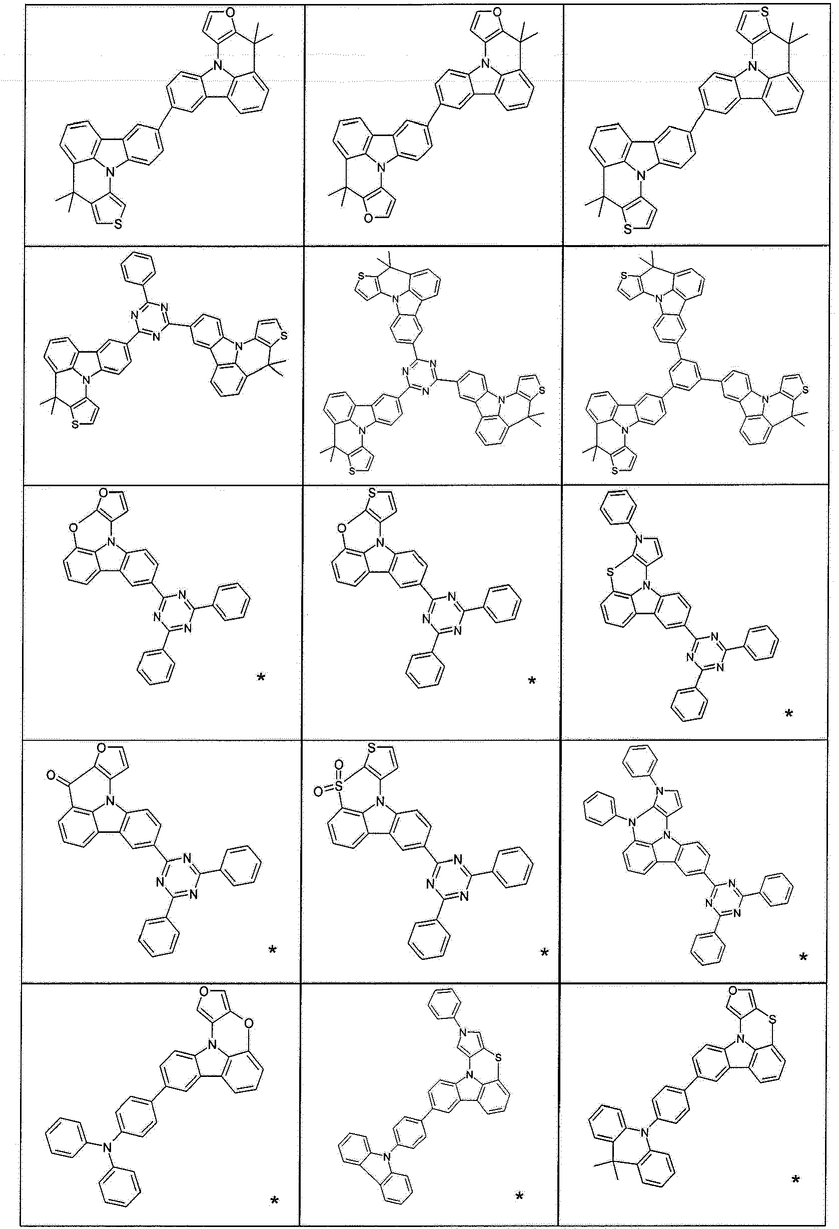

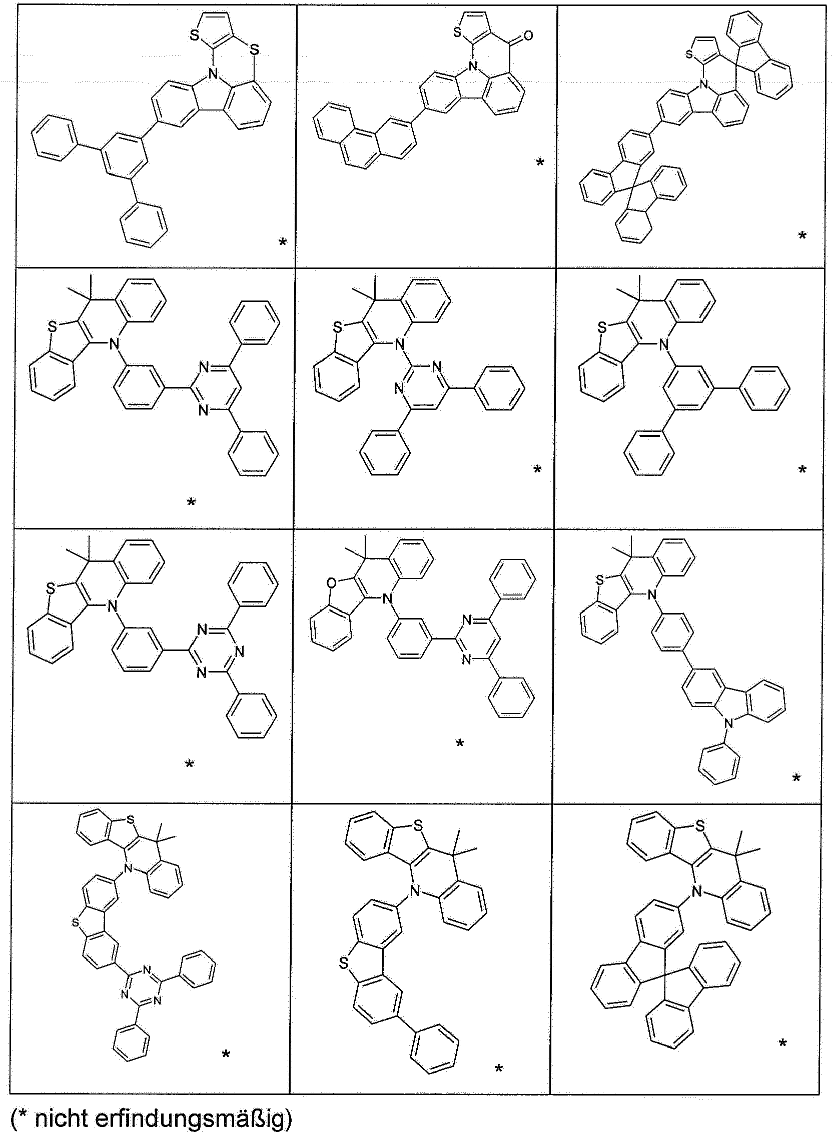

Die Synthese der erfindungsgemäßen Verbindungen ist in den folgenden Schemata gezeigt. In Schema 1a und 1b und in Schema 2 wird die Synthese von Verbindungen gezeigt, in denen Ar² und Ar³ für Phenylgruppen und Y² für eine Einfachbindung stehen. Als Edukt wird hierfür ein gegebenenfalls substituiertes Carbazol eingesetzt, welches in einer Ullmann-Kupplung, einer Buchwald-Kupplung oder einer anderen Kupplungsreaktion mit einem entsprechend substituierten Fünfring-Heteroaromaten umgesetzt wird. Die so erhaltenen erfindungsgemäßen Verbindungen können durch Reaktionssequenzen wie Bromierung und anschließende C-C- und C-N-Kupplungsreaktionen weiter funktionalisiert werden, wie auch in Schema 1 und 2 gezeigt. Je nach gewünschter Position der Bromsubstitution kann eine Cyclisierung über die Zwischenstufe eines tertiären Alkohols vor der Bromierung (Schema 1) oder nach der Bromierung (Schema 2) erfolgen. Durch den Ringschluss unter Säureeinfluss entsteht eine bivalente Brücke zwischen dem aromatischen Substituenten und dem Carbazol. Dabei eignet sich beispielsweise eine Carbonsäureestergruppe oder eine Acetylgruppe, welche dann in der Ringschlussreaktion zu einer Kohlenstoffbrücke umgesetzt werden kann (Schema 1 und 2). Dabei steht R in den Schemata für einen Substituenten, wie oben definiert.

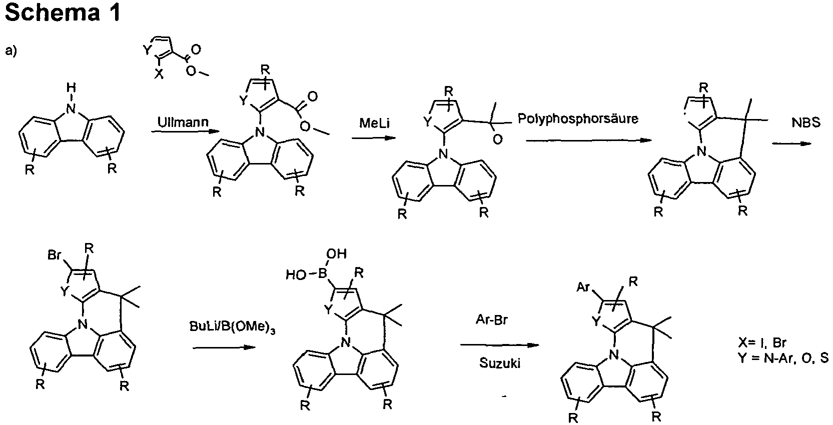

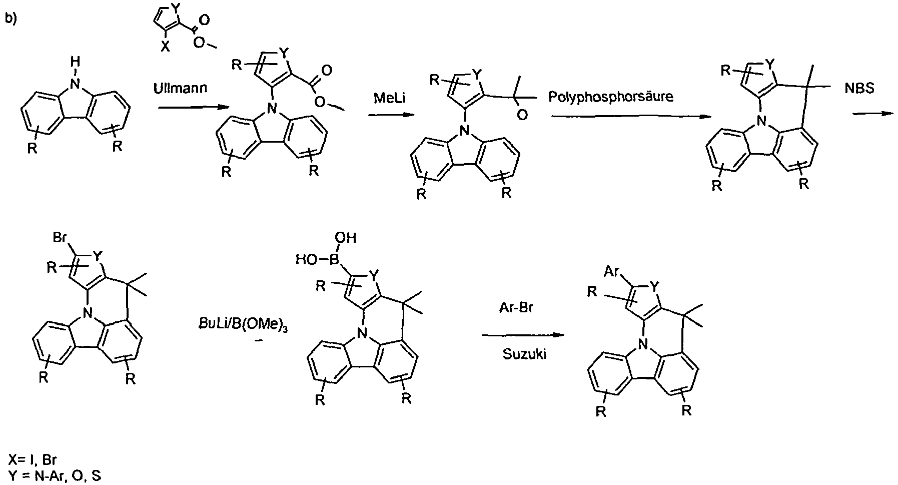

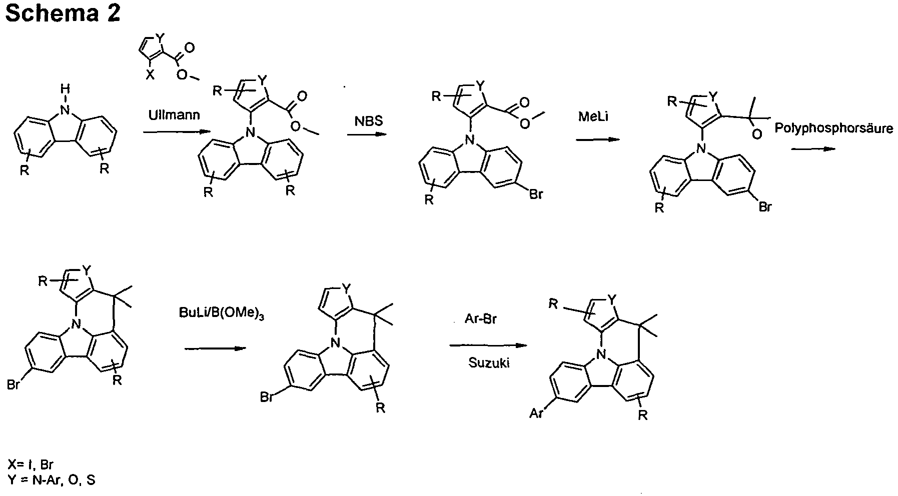

Die Einführung aromatischer Substituenten an der Brücke Y ist exemplarisch im folgenden Schema 3 gezeigt. Dabei wird der Carbonsäureester statt mit einer Alkyl-organischen Verbindung mit einer Aryl-organischen Verbindung, beispielsweise mit einer aromatischen Grignard-Verbindung, umgesetzt.

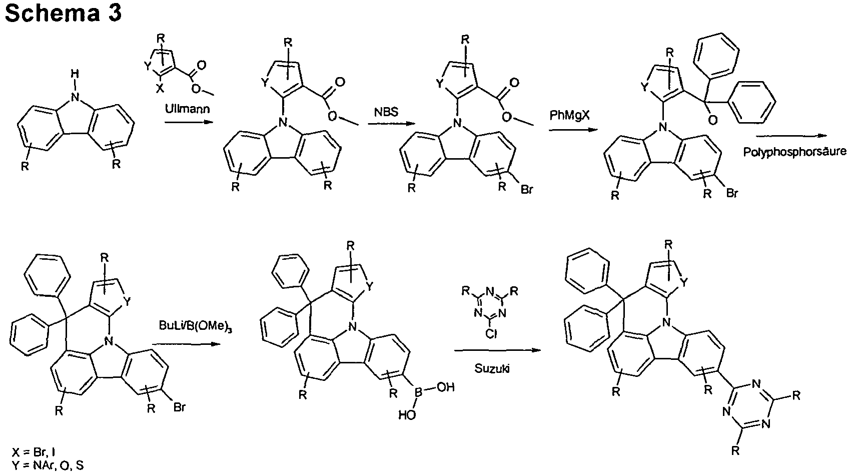

Weiterhin eignet sich eine Arylalkoholgruppe, welche dann in der Ringschlussreaktion zu einer Sauerstoffbrücke umgesetzt werden kann, oder eine Thiogruppe, welche dann in der Ringschlussreaktion zu einer Schwefelbrücke umgesetzt werden kann (Schema 4). Ebenso eignet sich eine Nitrogruppe oder Aminogruppe, welche dann in der Ringschlussreaktion zu einer Stickstoffbrücke umgesetzt werden kann (Schema 5). Die bivalente Brücke kann im weiteren Verlauf mit weiteren Resten substituiert werden, beispielsweise mit Alkyl- oder Arylgruppen. Die so hergestellte verbrückte Carbazolverbindung kann nun in einem weiteren Schritt funktionalisiert werden, beispielsweise halogeniert, bevorzugt bromiert.

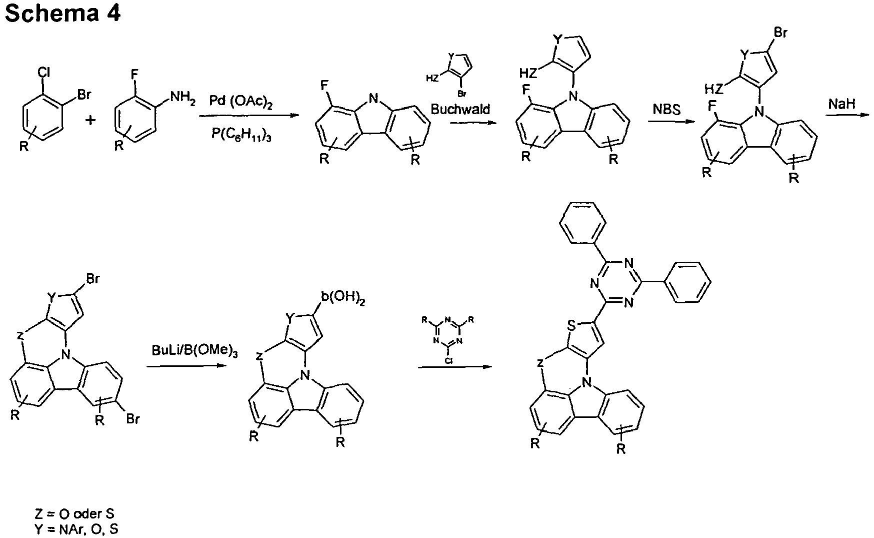

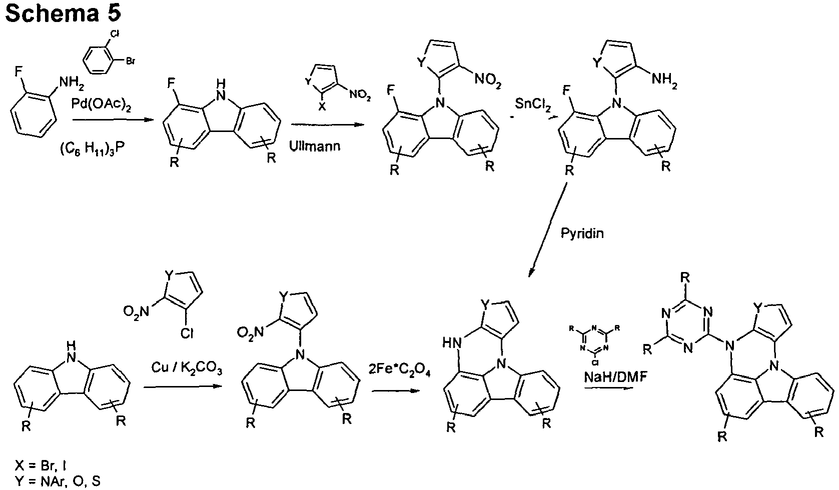

Die funktionalisierten, insbesondere bromierten Verbindungen stellen den zentralen Baustein für die weitere Funktionalisierung dar, wie in Schema 1 bis 5 dargestellt. So lassen sich diese funktionalisierten verbrückten Verbindungen leicht in entsprechende Boronsäuren überführen und beispielsweise durch Suzuki-Kupplung mit halogenierten Aromaten in weitere erfindungsgemäße Verbindungen gemäß Formel (1) umsetzen. Ebenso können andere Kupplungsreaktionen (z. B. Stille-Kupplung, Heck-Kupplung, Sonogashira-Kupplung, etc.) Verwendung finden. Kupplung mit Diarylaminen nach Hartwig-Buchwald führt zu Triarylamin-Derivaten. Entsprechend können aliphatische Amine, Carbazole, etc. als Substituenten eingeführt werden. Als Funktionalisierung kommen weiterhin Formyl-, Alkylcarbonyl- und Arylcarbonyl-Gruppen oder deren geschützte Analoga, z. B. in Form der entsprechenden Dioxolane, in Frage. Die bromierten Verbindungen können weiterhin lithiiert und durch Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden.

Verbindungen, in denen Y² statt für eine Einfachbindung für eine bivalente Gruppe steht, sind ganz analog zugänglich, indem statt Carbazol entsprechend Verbindungen als Edukt eingesetzt werden, die eine andere Gruppe Y² enthalten.

Ein weiterer Gegenstand der vorliegenden Erfindung ist daher ein Verfahren zur Herstellung einer Verbindung gemäß Formel (1) oder (2), umfassend die Reaktionsschritte:
a) Synthese des Grundgerüsts, welches statt einer Gruppe R eine reaktive Abgangsgruppe trägt; und
b) Einführung der Gruppe R, bevorzugt durch eine Kupplungsreaktion, beispielsweise Suzuki-Kupplung oder Hartwig-Buchwald-Kupplung.

Dabei ist die reaktive Abgangsgruppe bevorzugt ausgewählt aus Cl, Br, I, Boronsäure bzw. Boronsäurederivaten, Triflat oder Tosylat oder Y steht für NH, d. h. die reaktive Abgangsgruppe ist Wasserstoff, wenn eine Bindung zwischen N und R geknüpft wird.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird. Geeignete fluoreszierende und phosphoreszierende Dotanden sind unten im Zusammenhang mit den organischen Elektrolumineszenzvorrichtungen aufgeführt und sind auch für die erfindungsgemäßen Mischungen bevorzugt. Die weitere Verbindung kann auch ein Dotierstoff sein, wenn die erfindungsgemäße Verbindung als Lochtransport- oder Elektronentransportverbindung eingesetzt wird. Geeignete Dotierstoffe sind unten im Zusammenhang mit den organischen Elektrolumineszenzvorrichtungen aufgeführt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der oben ausgeführten erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bis (11) bzw. Formel (5a) bis (11d) als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bis (11) bzw. Formel (5a) bis (11d) als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bis (11) bzw. Formel (5a) bis (11d) als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bis (11) bzw. (5a) bis (11d) und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bis (11) bzw. (5a) bis (11d) bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bis (11) bzw. Formel (5a) bis (11d) als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bis (11) bzw. Formel (5a) bis (11d) eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, oder überbrückte Carbazol-Derivate, z. B. gemäß US 2009/0136779, WO 2010/050778 oder gemäß den nicht offen gelegten Anmeldungen DE 102009048791.3 oder DE 102010005697.9. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709 und WO 2011/032626 entnommen werden. Weiterhin eignen sich die Komplexe gemäß den nicht offen gelegten Anmeldungen DE 102009057167.1, EP 10006208.2 und DE 102010027317.1. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.
In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.
In einer weiteren bevorzugten Ausführungsform der Erfindung wird die Verbindung gemäß Formel (5a) bis (11d) als Elektronentransportmaterial in einer Elektronentransport- oder Elektroneninjektionsschicht eingesetzt. Dabei ist bevorzugt mindestens ein Substituent R oder R¹, insbesondere R, ausgewählt aus Strukturen der oben genannten Formeln (12) bis (33). Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. Liq (Lithiumhydroxychinolinat).
In nochmals einer weiteren bevorzugten Ausführungsform der Erfindung wird die Verbindung gemäß Formel (5a) bis (11d) in einer Lochblockierschicht eingesetzt. Dabei ist bevorzugt mindestens ein Substituent R oder R¹, insbesondere R, ausgewählt aus Strukturen der oben genannten Formeln (12) bis (33). Unter einer Lochblockierschicht wird eine Schicht verstanden, die auf Kathodenseite direkt an eine emittierende Schicht angrenzt, insbesondere in einer phosphoreszierenden Elektrolumineszenzvorrichtung.

Es ist weiterhin möglich, die Verbindung gemäß Formel (5a) bis (11d) sowohl in einer Lochblockierschicht bzw. Elektronentransportschicht als auch als Matrix in einer emittierenden Schicht zu verwenden. Dabei ist bevorzugt mindestens ein Substituent R oder R¹, insbesondere R, ausgewählt aus Strukturen der oben genannten Formeln (12) bis (33).
In nochmals einer weiteren Ausführungsform der Erfindung wird die Verbindung gemäß Formel (5a) bis (11d) in einer Lochtransportschicht bzw. in einer Elektronenblockierschicht bzw. Exzitonenblockierschicht eingesetzt. Dabei ist bevorzugt mindestens ein Substituent R oder R¹, insbesondere R, ausgewählt aus Strukturen der oben genannten Formeln (34) bis (47).
In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (5a) bis (11d) einsetzen.
Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.
Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).
Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.
Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.
Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.
Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen bzw. Verbindungen gemäß Formel (5a) bis (11d), eingesetzt als Matrixmaterial für fluoreszierende oder phosphoreszierende Emitter führen zu hohen Effizienzen sowie zu langen Lebensdauern. Dies gilt insbesondere, wenn die Verbindungen als Matrixmaterial für einen phosphoreszierenden Emitter eingesetzt werden.
2. Die erfindungsgemäßen Verbindungen bzw. Verbindungen gemäß Formel (5a) bis (11d) eignen sich nicht nur als Matrix für rot und grün phosphoreszierende Verbindungen, sondern insbesondere auch für blau phosphoreszierende Verbindungen.
3. Im Gegensatz zu vielen Verbindungen gemäß dem Stand der Technik, die der teilweisen oder vollständigen pyrolytischen Zersetzung bei Sublimation unterliegen, weisen die erfindungsgemäßen Verbindungen eine hohe thermische Stabilität auf.
4. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
5. Auch bei Verwendung als Elektronentransportmaterial bzw. als Lochtransportmaterial führen die erfindungsgemäßen Verbindungen zu guten Eigenschaften in Bezug auf die Effizienz, die Lebensdauer und die Betriebsspannung von organischen Elektrolumineszenzvorrichtungen.
Diese oben genannten Vorteile gehen nicht mit einer Verschlechterung der weiteren elektronischen Eigenschaften einher.
Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Als Ausgangspunkt kann z. B. 3-Bromothiophen-2-carbonsäuremethylester (Synlett 2004, 6, 1113-1116) dienen. Die Zahlen bei den literaturbekannten Edukten beziehen sich auf die CAS-Nummer.

### Beispiel 1a: 10-Brom-8,8-dimethyl-8H-9-thia-11b-aza-cyclopenta[a]-fluoranthen (Verbindung 1a)

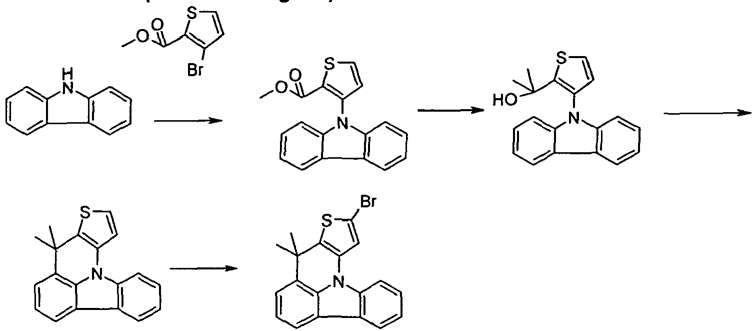

### Schritt 1: 3-Carbazol-9-yl-thiophen-2-carbosäuremethylester

102 g (420 mmol) 3-Phenyl-9H-carbazol, 92 g (420 mmol) 3-Bromthiophen-2-carbonsäuremethylester, 24 g (375 mmol) Kupferpulver, 104 g (757 mmol) Kaliumcarbonat und 11 g (42 mmol) 18-Krone-6 werden unter Schutzgas in 1200 ml DMF vorgelegt und 86 h auf 130 °C erhitzt. Anschließend wird die Mischung eingeengt, heiß mit Heptan ausgerührt und chromatographisch gereinigt (Heptan, Dichloromethan 1:1). Das Produkt wird mit Hexan heiß ausgerührt und der Feststoff wird isoliert. Ausbeute: 121 g (397 mmol), 65 % d. Th., Reinheit nach ¹H-NMR ca. 97 %.

### Schritt 2: 2-(3-Carbazol-9-yl-thiophen-2-yl)-propan-2-ol

85 g (277 mmol) 3-Carbazol-9-yl-thiophen-2-carbosäuremethylester werden in 1700 mL getrocknetem THF gelöst und entgast. Es wird auf -78 °C gekühlt und innerhalb von 40 min mit 740 ml (1110 mmol) Methyllithium versetzt. Man lässt innerhalb 1 h bis auf -40 °C erwärmen und kontrolliert die Umsetzung via DC. Nach vollständiger Umsetzung wird bei -30 °C vorsichtig mit MeOH gequencht. Die Reaktionslösung wird auf 1/3 des Volumens eingeengt, mit 1 L Methylenchlorid versetzt, gewaschen, die organische Phase über MgSO₄ getrocknet und eingeengt. Ausbeute: 96 g (249 mmol), 90 % d. Th., Reinheit nach ¹H-NMR ca. 97 %.

### Schritt 3: 8,8-Dimethyl-8H-9-thia-11b-aza-cyclopenta[a]fluoranthen

20 g (43.6 mmol) 2-(3-Carbazol-9-yl-thiophen-2-yl)-propan-2-ol werden in 1.2 L entgastem Toluol gelöst und mit einer Suspension aus 52 g Polyphosphorsäure und 36 mL Methansulfonsäure versetzt und 1 h auf 60 °C erhitzt. Der Ansatz wird abgekühlt und mit Wasser versetzt. Es fällt ein Feststoff aus, der mit Methylenchlorid/THF (1:1) gelöst wird. Die Lösung wird mit 20%iger NaOH vorsichtig alkalisiert, die Phasen werden getrennt und über MgSO₄ getrocknet. Der erhaltene Feststoff wird mit Heptan ausgerührt. Ausbeute: 12 g (41 mmol), 80% d. Th., Reinheit nach ¹H-NMR ca. 93 %.

### Schritt 4: 10-Brom-8,8-dimethyl-8H-9-thia-11b-aza-cyclopenta[a]-fluoranthen

60 g (207 mmol) 8,8-Dimethyl-8H-9-thia-11b-aza-cyclopenta[a]fluoranthen werden in 2 L DMF auf -10 °C gekühlt und portionsweise mit 37.3 g (207 mmol) NBS versetzt. Anschließend lässt man auf Raumtemperatur kommen und rührt 6 h bei dieser Temperatur. Dann wird die Mischung mit 500 mL Wasser versetzt und mit CH₂Cl₂ extrahiert. Die organische Phase wird über MgSO₄ getrocknet und die Lösungsmittel im Vakuum entfernt. Das Produkt wird mit Toluol heiß ausgerührt und der Feststoff wird isoliert. Ausbeute: 73 g (201 mmol), 97 % d. Th., Reinheit nach ¹H-NMR ca. 98 %.

Analog werden die Verbindungen **1b** - **1n** erhalten:

| **Bsp.** | **Edukt 1** | **Edukt 1** | **Produkt** | **Ausbeute** |
|---|---|---|---|---|
| **1b** | 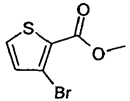 | 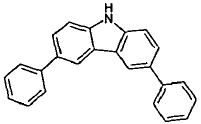 | 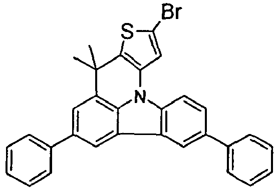 | 95% |
| | 26137-08-6 | 56525-79-2 | | |
| **1c** | 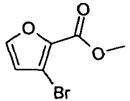 | 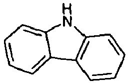 | 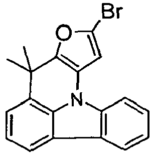 | 69% |
| | 59862-77-0 | | | |
| **1d** | 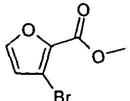 | 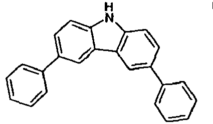 | 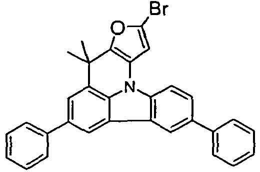 | 81% |
| | 59862-77-0 | 56525-79-2 | | |
| **1e** | 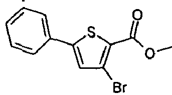 | 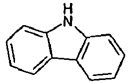 | 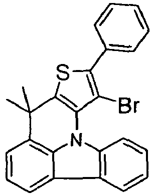 | 61% |
| | 478028-23-8 | | | |
| **1f*** | 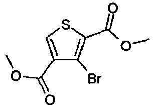 | 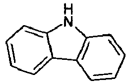 | 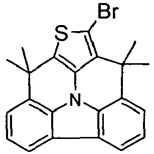 | 62% |
| | 24647-86-7 | | | |
| **1g** | 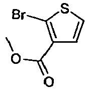 | 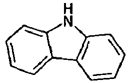 | 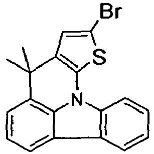 | 63% |
| | 76360-43-5 | | | |
| **1g** | 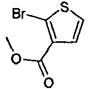 | 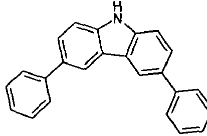 | 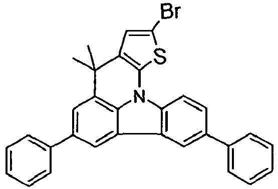 | 79% |
| | 76360-43-5 | 56525-79-2 | | |
| **1h** | 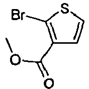 | 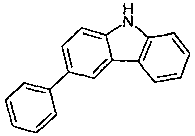 | 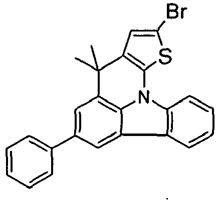 | 56% |
| | 76360-43-5 | 103012-26-6 | | |
| **1j** | 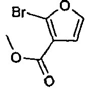 | 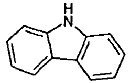 | 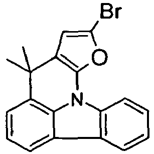 | 62% |
| | 197846-06-3 | | | |
| **1i** | 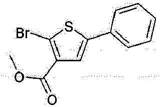 | 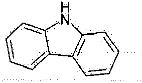 | 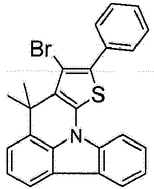 | 71 % |
| | 923010-50-8 | | | |
| **1k*** | 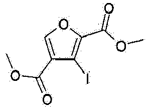 | 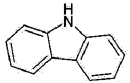 | 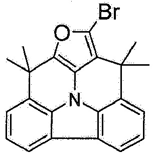 | 56 % |
| | 35189-81-2 | | | |
| **1l** | 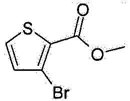 | 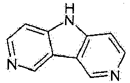 | 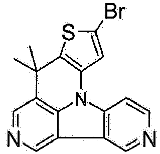 | 83 % |
| | 26137-08-6 | 244-78-0 | | |
| **1m*** | 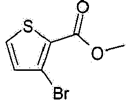 | 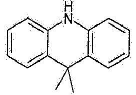 | 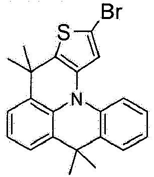 | 65 % |
| | 26137-08-6 | 6267-02-3 | | |
| **1n*** | 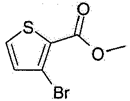 | 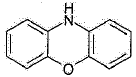 | 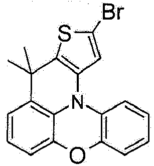 | 62 % |
| | 26137-08-6 | 135-67-1 | | |

| | | | | |
|---|---|---|---|---|
| (* nicht erfindungsmäßig) | | | | |

### Beispiel 2a: 3-Brom-8,8-dimethyl-8H-9-thia-11b-aza-cyclopenta[a]-fluoranthen (Verbindung 2a)

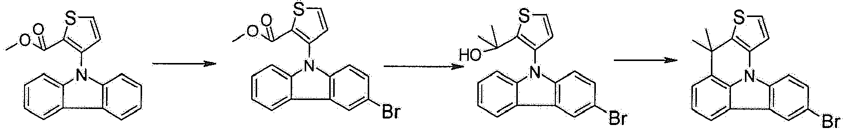

### Schritt 1: 3-(3-Bromcarbazol-9-yl)-thiophen-2-carbosäuremethylester

63.5 g (207 mmol) 8,8-Dimethyl-8H-9-thia-11b-azacyclopenta[a]-fluoranthen werden in 2 L DMF auf -10 °C gekühlt und portionsweise mit 37.3 g (207 mmol) NBS versetzt. Anschließend lässt man auf Raumtemperatur kommen und rührt 6 h bei dieser Temperatur. Dann wird die Mischung mit 500 mL Wasser versetzt und mit CH₂Cl₂ extrahiert. Die organische Phase wird über MgSO₄ getrocknet und die Lösungsmittel im Vakuum entfernt. Das Produkt wird mit Toluol heiß ausgerührt und der Feststoff wird isoliert. Ausbeute: 72 g (186 mmol), 90 % d. Th., Reinheit nach ¹H-NMR ca. 97 %.

### Schritt 2: 2-[3-(3-Bromcarbazol-9-yl)-thiophen-2-yl]-propan-2-ol

106 g (277 mmol) 3-(3-Bromcarbazol-9-yl)-thiophen-2-carbonsäuremethylester werden in 1700 mL getrocknetem THF gelöst und entgast. Es wird auf -78 °C gekühlt und innerhalb von 40 min mit 740 ml (1110 mmol) Methyllithium versetzt. Man lässt innerhalb 1 h bis auf -40 °C erwärmen und kontrolliert die Umsetzung via DC. Nach vollständiger Umsetzung wird bei -30 °C vorsichtig mit MeOH gequencht. Die Reaktionslösung wird auf 1/3 des Volumens eingeengt, mit 1 L Methylenchlorid versetzt, gewaschen, die organische Phase über MgSO₄ getrocknet und eingeengt. Ausbeute: 97 g (251mmol), 91 % d. Th., Reinheit nach ¹H-NMR ca. 97 %.

### Schritt 3: 8,8-Dimethyl-8H-9-thia-11b-aza-cyclopenta[a]fluoranthen

20 g (43.6 mmol) 2-(3-Bromcarbazol-9-yl-thiophen-2-yl)-propan-2-ol werden in 1.2 L entgastem Toluol gelöst und mit einer Suspension aus 52 g Polyphosphorsäure und 36 mL Methansulfonsäure versetzt und für 1 h auf 60 °C erhitzt. Der Ansatz wird abgekühlt und mit Wasser versetzt. Es fällt ein Feststoff aus, der mit Methylenchlorid/THF (1:1) gelöst wird. Die Lösung wird mit 20%iger NaOH vorsichtig alkalisiert, die Phasen werden getrennt und über MgSO₄ getrocknet. Der erhaltene Feststoff wird aus Heptan ausgerührt. Ausbeute: 12 g (41 mmol), 80% d. Th., Reinheit nach ¹H-NMR ca. 93 %.

### Schritt 4: 3-Brom-8,8-dimethyl-8H-9-thia-11b-aza-cyclopenta[a]-fluoranthen

80 g (207 mmol) 8,8-Dimethyl-8H-9-thia-11b-aza-cyclopenta[a]fluoranthen wird in 2 L DMF auf -10 °C gekühlt und portionsweise mit 37.3 g (207 mmol) NBS versetzt. Anschließend lässt man auf Raumtemperatur kommen und rührt 6 h bei dieser Temperatur. Dann wird die Mischung mit 500 mL Wasser versetzt und mit CH₂Cl₂ extrahiert. Die organische Phase wird über MgSO₄ getrocknet und die Lösungsmittel im Vakuum entfernt. Das Produkt wird mit Toluol heiß ausgerührt und der Feststoff wird isoliert. Ausbeute: 70 g (190 mmol), 92 % d. Th., Reinheit nach ¹H-NMR ca. 98 %.

Analog werden die Verbindungen **2b - 2e** erhalten:

| **Bsp.** | **Edukt 1** | **Produkt** | **Ausbeute** |
|---|---|---|---|
| **2b** | 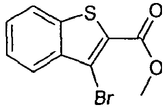 | 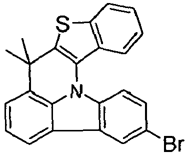 | 59 % |
| | 34128-30-8 | | |
| **2c** | 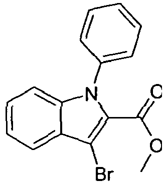 | 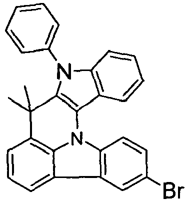 | 74% |
| | 1248548-62-0 | | |
| **2d** | 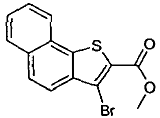 | 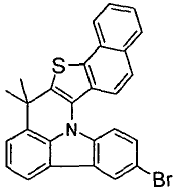 | 65% |
| | 145429-99-8 | | |
| **2e** | 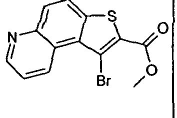 | 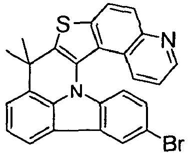 | 51% |
| | 1188365-72-1 | | |

### Beispiel 3a: 8,8-Dimethyl-8H-9-thia-11b-aza-cyclopenta[a]fluoranthen-10-boronsäure (Verbindung 3a)

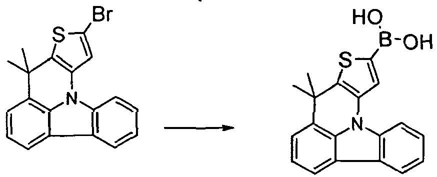

85 g (233 mmol) 10-Brom-8,8-dimethyl-8H-9-thia-11b-aza-cyclopenta[a]-fluoranthen werden in 1400 mL trockenem THF gelöst, bei -70 °C 121 mL (303 mmol) einer 2.5 M Lösung von n-Butyllithium in Cyclohexan zugetropft, nach 1 h 33 mL Trimethylborat (302 mmol) zugetropft, innerhalb 1 h auf Raumtemperatur kommen gelassen, das Lösungsmittel entfernt und der Rückstand, der nach ¹H-NMR einheitlich ist, ohne weitere Reinigung in die Folgereaktion eingesetzt. Die Ausbeute beträgt 69 g (207 mmol), entsprechend 90 % der Theorie.

Analog werden die Verbindungen **3b - 3o** erhalten:

| **Bsp.** | **Edukt 1** | **Produkt** | **Ausbeute** |
|---|---|---|---|
| **3b** | 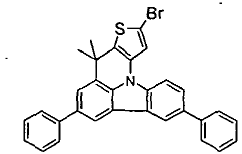 | 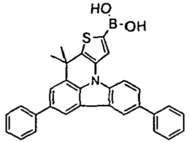 | 86 % |
| **3c** | 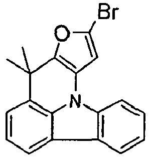 | 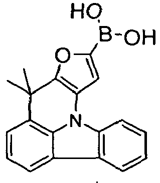 | 79% |
| **3d** | 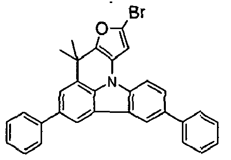 | 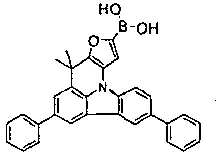 | 83% |
| **3e*** | 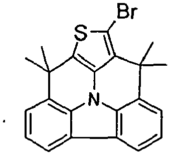 | 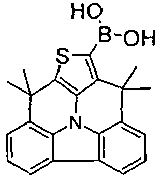 | 83 % |
| **3f** | 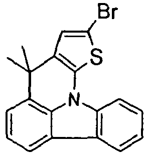 | 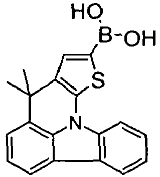 | 77% |
| **3g** | 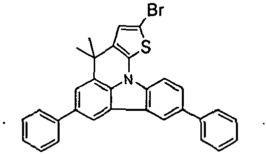 | 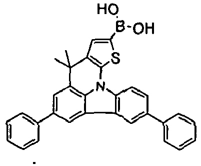 | 86% |
| **3h** | 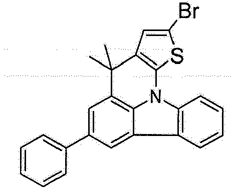 | 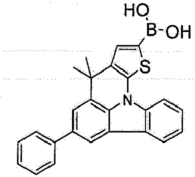 | 80% |
| **3j** | 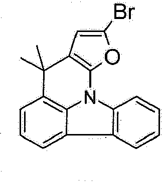 | 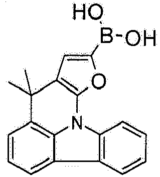 | 69% |
| **3i** | 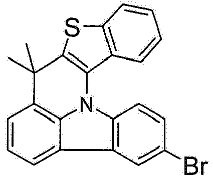 | 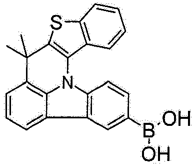 | 82% |
| **3k** | 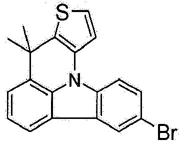 | 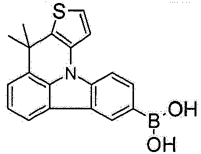 | 64% |
| **3l** | 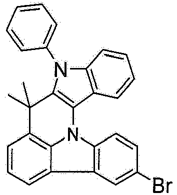 | 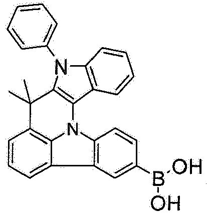 | 56% |
| **3m*** | 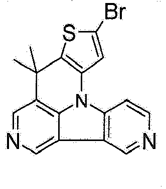 | 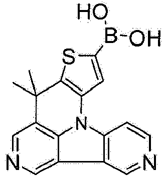 | 80 % |
| **3n*** | 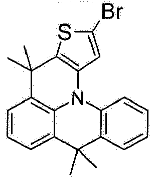 | 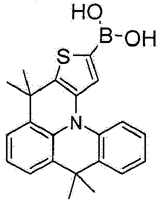 | 75 % |
| **3o*** | 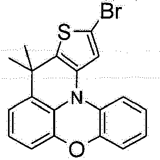 | 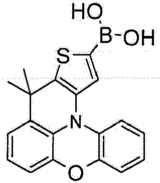 | 78 % |

| | | | |
|---|---|---|---|
| (* nicht erfindungsmäßig) | | | |

### Beispiel 4a: 10-(4,6-Diphenyl-[1,3,5]triazin-2-yl)-8,8-dimethyl-8H-9-thia-11b-aza-cyclopenta[a]fluoranthen (Verbindung 4a)

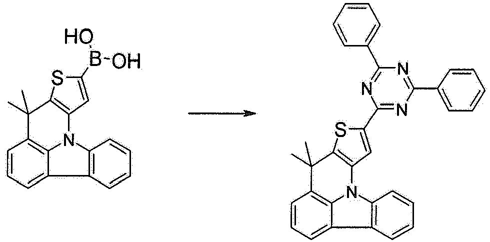

36.6 g (110.0 mmol) 8,8-Dimethyl-8H-9-thia-11b-aza-cyclopenta[a]-fluoranthen-10-boronsäure, 29.5 g (110.0 mmol) 2-Chlor-4,6-diphenyl-1,3,5-triazin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum sublimiert, Reinheit beträgt 99.9 %. Die Ausbeute beträgt 45 g (88 mmol), entsprechend 80 % der Theorie.

Analog werden die Verbindungen **4b - 4r** erhalten:

| **Bsp.** | **Edukt 1** | **Produkt** | **Produkt** | **Ausbeute** |
|---|---|---|---|---|
| **4b** | 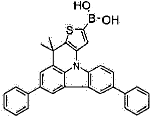 | 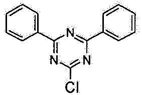 | 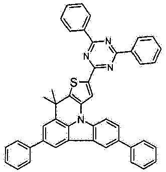 | 83 % |
| | | 3842-55-5 | | |
| **4c** | 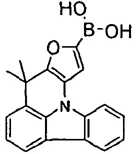 | 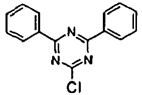 | 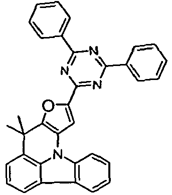 | 87% |
| | | 3842-55-5 | | |
| **4d** | 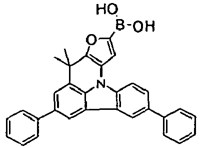 | 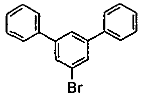 | 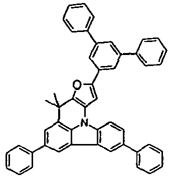 | 80% |
| | | 103068-20-8 | | |
| **4e** | 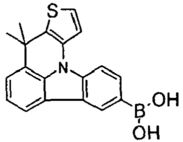 | 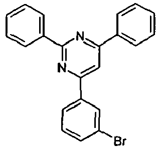 | 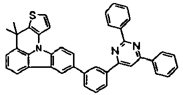 | 83 % |
| | | 864377-28-6 | | |
| **4f** | 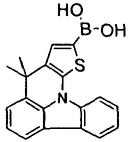 | 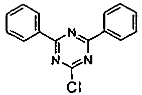 | 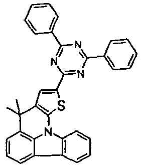 | 67% |
| | | 3842-55-5 | | |
| **4g** | 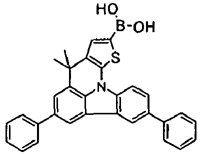 | 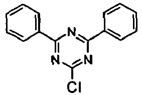 | 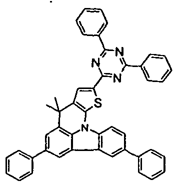 | 86% |
| | | 3842-55-5 | | |
| **4h** | 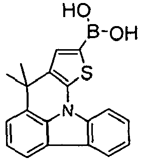 | 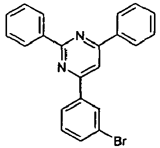 | 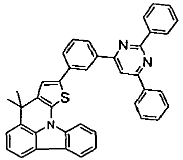 | 80 % |
| | | 864377-28-6 | | |
| **4j** | 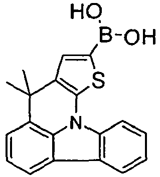 | 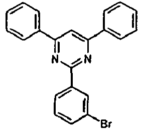 | 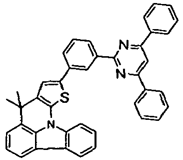 | 69% |
| | | 864377-22-0 | | |
| **4i** | 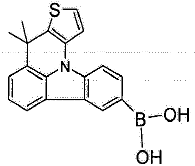 | 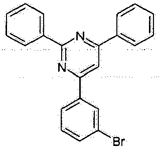 | 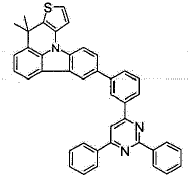 | 82% |
| | | 864377-28-6 | | |
| **4k** | 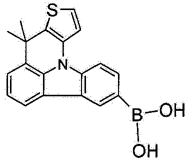 | 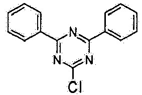 | 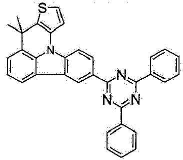 | 75% |
| **4l** | 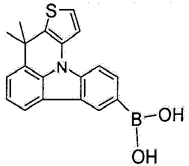 | 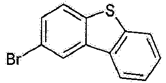 | 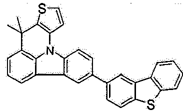 | 87% |
| | | 22439-61-8 | | |
| **4m** | 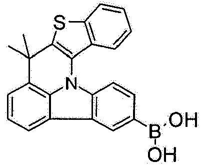 | 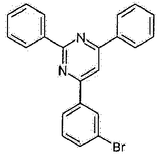 | 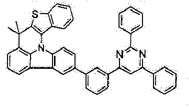 | 65% |
| | | 864377-28-6 | | |
| **4n** | 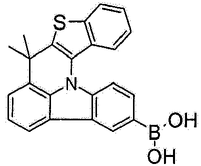 | 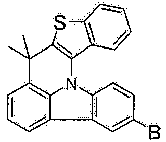 | 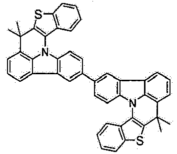 | 69% |
| **4o*** | 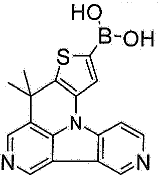 | 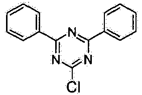 | 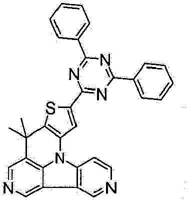 | 73 % |
| | | 3842-55-5 | | |
| **4p*** | 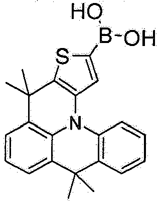 | 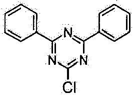 | 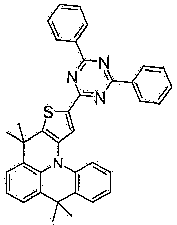 | 78 % |
| | | 3842-55-5 | | |
| **4q*** | 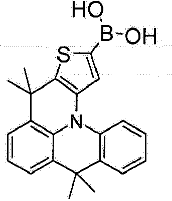 | 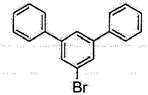 | 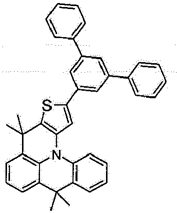 | 68 % |
| | | 103068-20-8 | | |
| **4r*** | 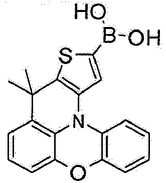 | 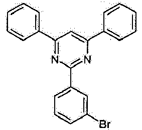 | 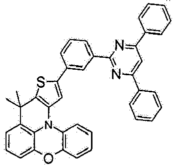 | 71 % |
| | | 864377-22-0 | | |

| | | | | |
|---|---|---|---|---|
| (* nicht erfindungsmäßig) | | | | |

### Beispiel 5: 8,8-Dimethyl-6-[4-(1-phenyl-1H-benzoimidazol-2-yl)-phenyl]-8H-9-thia-11b-aza-cyclopenta[a]fluoranthen (Verbindung 5)

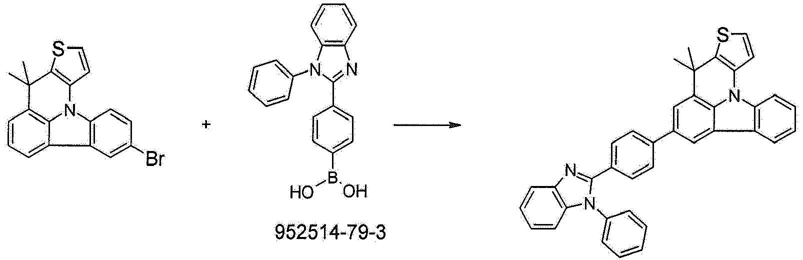

Ein entgaste Suspension von 10.3 g (28 mmol) 6-Brom-8,8-dimethyl-8H-indolo[3,2,1,-de]acridin und 9,42 g (30 mmol) Benzimidazolboronsäure und 7.8 g (31.5 mmol) Kaliumphosphat-hydrat in einem Gemisch aus 7.5 ml Dioxan, 15 ml Toluol und 18 ml Wasser wird unter gutem Rühren mit 0.27 g (0.9 mmol) Tri-o-tolylphosphin und dann mit 33.5 mg (0.15 mmol) Palladium(II)acetat versetzt. Nach 5 h Erhitzen unter Rückfluss lässt man die Mischung erkalten. Der Niederschlag wird abgesaugt, dreimal mit 10 ml Ethanol / Wasser (1:1, v:v) und dreimal mit 5 ml Ethanol gewaschen, anschließend im Vakuum getrocknet und aus Dioxan umkristallisiert. Ausbeute: 12.7 g (22,9 mmol), 82 % d. Th., Reinheit nach ¹H-NMR ca. 99.9 %.

### Beispiel 6a: 8,8-Dimethyl-3-(9-phenyl-9H-carbazol-3-yl)-8H-9-thia-11b-aza-cyclopenta[a]fluoranthen (Verbindung 6a)

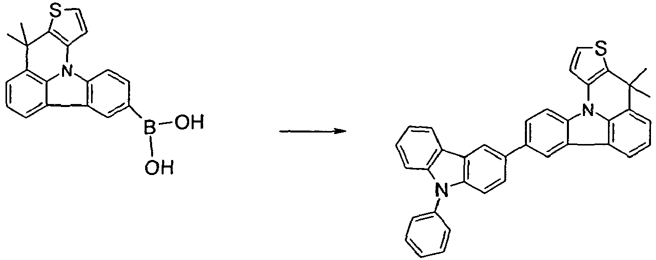

36.6 g (110 mmol) 8,8-Dimethyl-8H-9-thia-11b-azacyclopenta[a]-fluoranthen-3-boronsäure, 35 g (110 mmol) 3-Brom-9-phenyl-9H-carbazol und 9.7 g (92 mmol) Natriumcarbonat werden in 350 mL Toulol, 350 mL Dioxan und 500 mL Wasser suspendiert. Zu dieser Suspension werden 913 mg (3.0 mmol) Tri-o-tolylphosphin und 112 mg (0.5 mmol) Palladium-(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus CH₂Cl₂/*iso-*Propanol umkristallisiert und abschließend im Hochvakuum sublimiert. Ausbeute: 52,4 g (100 mmol), 90% d. Th., Reinheit nach HPLC 99.9%.

Analog werden die Verbindungen **6b** - **6j** erhalten:

| **Bsp.** | **Edukt 1** | **Produkt** | **Produkt** | **Ausbeute** |
|---|---|---|---|---|
| **6b** | 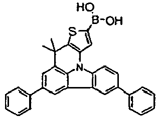 | 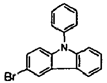 | 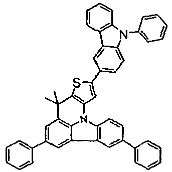 | 81 % |
| | | 1153-85-1 | | |
| **6c** | 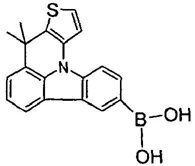 | 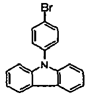 | 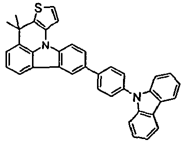 | 84% |
| | | 57102-42-8 | | |
| **6d** | 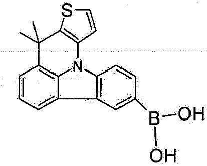 | 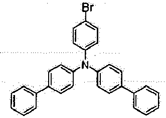 | 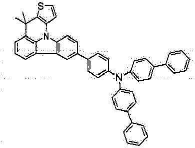 | 79% |
| | | 499128-71-1 | | |
| **6e** | 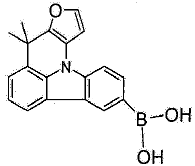 | 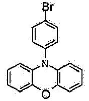 | 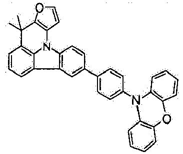 | 83 % |
| | | 71041-21-9 | | |
| **6f** | 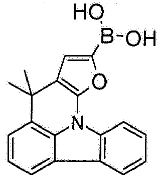 | 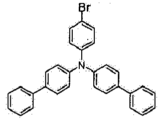 | 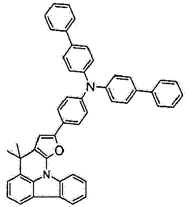 | 67% |
| | | 499128-71-1 | | |
| **6g** | 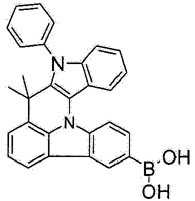 | 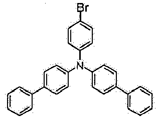 | 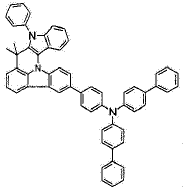 | 77% |
| | | 499128-71-1 | | |
| **6h*** | 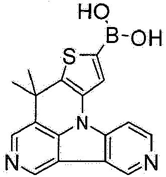 | 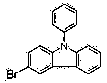 | 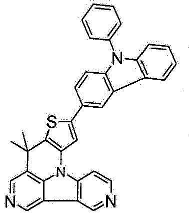 | 71 % |
| | | 1153-85-1 | | |
| **6i*** | 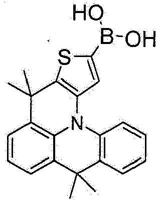 | 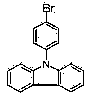 | 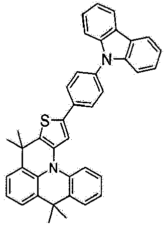 | 75 % |
| | | 57102-42-8 | | |
| **6j*** | 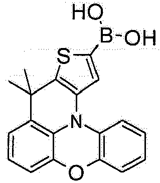 | 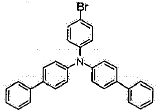 | 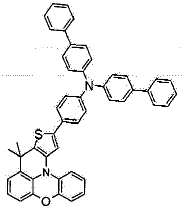 | 82 % |
| | | 499128-71-1 | | |

| | | | | |
|---|---|---|---|---|
| (* nicht erfindungsmäßig) | | | | |

### Beispiel 7: Biphenyl-4-yl-(9,9-dimethyl-9H-fluoren-2-yl)-[4-(8,8-di-methyl-8H-9-thia-11b-aza-cyclopenta[a]fluoranthen-3-yl)-phenyl]-amin (Verbindung 7)

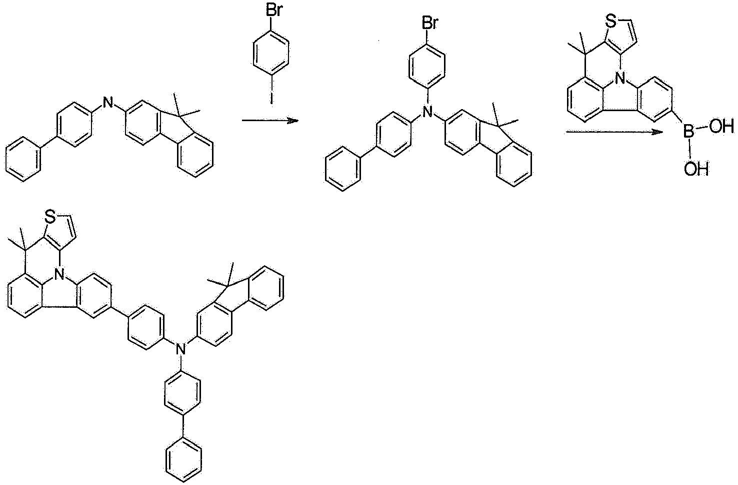

### Schritt 1: Biphenyl-4-yl-(4-bromphenyl)-(9,9-dimethyl-9H-fluoren-2-yl)amin

Eine entgaste Lösung von 490 mg (0.16 mmol) Kupfer(I)chlorid und 906 mg (5 mmol) 1,10-Phenanthrolin in 100 mL Toluol wird 1 h mit N₂ gesättigt und auf 130 °C erhitzt. Anschließend wird die Lösung mit 18 g (50 mmol) *N*-[1,1'-Biphenyl]-4-yl-9,9-dimethyl-9*H*-fluoren-2-amin und 14 g (50 mmol) 1-Brom-4-iodbenzol versetzt und 2 h auf 180 °C erhitzt. Nach Abkühlen wird die Mischung mit 180 mL Wasser versetzt, die organische Phase abgetrennt und das Lösungsmittel im Vakuum entfernt. Das Produkt wird aus *n*-Hexan umkristallisiert. Ausbeute: 15 g (29 mmol), 58% d. Th., Reinheit nach ¹H-NMR ca. 98%.

### Schritt 2: Biphenyl-4-yl-(9,9-dimethyl-9H-fluoren-2-yl)-[4-(8,8-dimethyl-8H-9-thia-11b-aza-cyclopenta[a]fluoranthen-3-yl)-phenyl]-amin

Die Verbindung wird nach der gleichen Vorschrift wie Beispiel 6a durch Umsetzung der entsprechenden 8,8-Dimethyl-8H-9-thia-11b-azacyclo-penta[a]fluoranthen-3-boronsäure mit 56.8 g (110 mmol) Biphenyl-4-yl-(4-bromphenyl)-(9,9-dimethyl-9*H*-fluoren-2-yl)amin synthetisiert. Der Rückstand wird aus Essigsäureethylester/Heptan umkristallisiert und abschließend im Hochvakuum sublimiert. Ausbeute: 57 g (79 mmol), 72% d. Th., Reinheit nach HPLC 99.9%.

### Beispiel 8 (nicht erfindungsmäßig): 5-[3-(4,6-Diphenyl-pyrimidin-2-yl)-phenyl]-10,10-dimethyl-5,5a,9a,10-tetrahydro-11-thia-5-aza-benzo[b]fluoren (Verbindung 8)

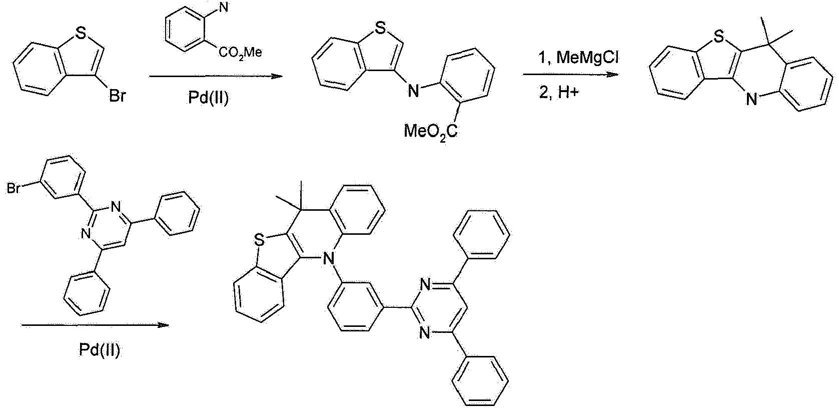

### Schritt 1: 2-(Benzo[b]thiophen-3-ylamino)-benzoesäuremethylester

40 g (187.7 mmol) 3-Brombenzothiophen, 24.25 mL (187.7 mmol) 2-Methylanthranilat, und 122 g (375 mmol) Cs₂CO₈ werden in 600 mL Toluol suspendiert. Zu dieser Suspension werden 2.10 g (9.38 mmol) Palladiumacetat und 3.69 g Xantphos (18.77 mmol) gegeben. Die Reaktionsmischung wird 24 h unter Rückfluss erhitzt. Nach Erkalten wird die Mischung eingeengt und anschließend zwischen Essigester und Wasser verteilt. Die organische Phase dreimal mit Wasser gewaschen und über Na₂SO₄ getrocknet, einrotiert und anschließend zur Trockene eingeengt. Der Rückstand wird aus Heptan umkristallisiert. Ausbeute: 32 g (60 %)

### Schritt 2: 10,10-Dimethyl-5,5a,9a,10-tetrahydro-11-thia-5-aza-benzo[b]-fluoren

25.9 g (105 mmol) Wasserfreies Cer(III)-chlorid werden in 400 mL trockenem THF vorgelegt. Zu dieser Lösung werden 30 g (105 mmol) 2-(Benzo[b]thiophen-3-ylamino)-benzoesäuremethylester portionsweise zudosiert und 1 h gerührt. Die Reaktionsmischung wird abgekühlt und bei 5 °C 140 mL (420 mmol) Methylmagnesiumchlorid-Lösung (3 mol/L in THF) innerhalb von 40 min zugetropft. Nach einer Stunde wird das Reaktionsgemisch vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 29.5 g (95 %)

35.60 g (309 mmol) Polyphosphorsäure und 20 mL (309 mmol) Methansulfonsäure werden in 300 mL CH₂Cl₂ vorgelegt. Zu dieser Lösung werden 25 g (88 mmol) 2-[2-(Benzo[b]thiophen-3-ylamino)-phenyl]-propan-2-ol in CH₂Cl₂-Lösung (100 mL) innerhalb von 30 min zugetropft und 1 h bei Raumtemperature gerührt. Das Reaktionsgemisch wird abgekühlt, vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 20 g 10,10-Dimethyl-5,5a,9a,10-tetrahydro-11-thia-5-aza-benzo[b]fluoren (85 %).

### Schritt 3: 5-[3-(4,6-Diphenyl-pyrimidin-2-yl)-phenyl]-10,10-dimethyl-5,5a,9a,10-tetrahydro-11-thia-5-aza-benzo[b]fluoren

18 g (68 mmol) 10,10-Dimethyl-5,5a,9a,10-tetrahydro-11-thia-5-aza-benzo[b]fluoren, 28.9 g (75 mmol) 2-(3-Bromphenyl)-4,6-diphenylpyrimidin und 19.6 g NaOtBu (203 mmol) werden in 500 mL p-Xylol suspendiert. Zu dieser Suspension werden 0.3 g (1.36 mmol) Pd(OAc)₂ und 2.7 mL eine 1M Tri-tert-butylphosphin Lösung gegeben. Die Reaktionsmischung wird 16 h unter Rückfluss erhitzt. Nach Erkalten wird die organische Phase abgetrennt dreimal mit 200 mL Wasser gewaschen und anschließend zur Trockene eingeengt. Der Rückstand wird mit Toluol heiß extrahiert und aus Toluol umkristallisiert und abschließend im Hochvakuum sublimiert, Reinheit beträgt 99.9%.

### Beispiel 9 (nicht erfindungsmäßig): 5,10-Bis-biphenyl-4-yl-11,11-dimethyl-10,11-dihydro-5H-indolo[3,2-b]chinolin (Verbindung 9)

### Schritt 1: 2-(1-Biphenyl-4-yl-1H-indol-3-ylamino)-benzoesäuremethyl-ester

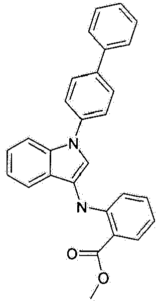

25 g (213 mmol) 1H-Indol, 89.6 g (320 mmol) Monoiodbiphenyl und 100 g K₃PO₄ werden in 1L Toluol suspendiert. Zu dieser Suspension werden 16.3 g (85 mmol) Cul und 7,5 g N,N'-Dimethylendiamin (85 mmol) gegeben. Die Reaktionsmischung wird 48 h unter Rückfluss erhitzt. Nach Erkalten wird der Niederschlag über einen Faltenfilter abfiltriert. Die Reaktionslösung wurde im Anschluss zwischen Essigester und Wasser verteilt, die organische Phase dreimal mit Wasser gewaschen, über Na₂SO₄ getrocknet, einrotiert, die organische Phase abgetrennt, dreimal mit 200 mL Wasser gewaschen und anschließend zur Trockene eingeengt. Das zurückgebliebene schwarzgrüne Öl wurde mit Heptan:Toluol über Kieselgel filtriert. Der eingedampfte Filtrat-Rückstand wurde aus Methanol umkristallisiert. Ausbeute: 41 g 1-Biphenyl-4-yl-1H-indol (70%).

40 g (149 mmol) 1-Biphenyl-4-yl-1H-indol werden in 500 mL Dichlormethan vorgelegt. Anschließend tropft man unter Lichtausschluss bei 0 °C eine Lösung aus 26.4 g (149 mmol) NBS in 200 ml Dichloromethan hinzu, lässt auf Raumtemperatur kommen und rührt 4 h weiter. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 49.1 g (95 %)

49 g (141 mmol) 1-Biphenyl-4-yl-3-brom-1H-indol, 18 mL (141 mmol) 2-Methylanthranilat und 91.7 g (281 mmol) Cs₂CO₃ werden in 800 mL Toluol suspendiert. Zu dieser Suspension werden 0.8 g (3.52 mmol) Palladiumacetat und 4 g Xantphos (7.04 mmol) gegeben. Die Reaktionsmischung wird 24 h unter Rückfluss erhitzt. Nach Erkalten wird die Mischung eingeengt und anschließend zwischen Essigester und Wasser verteilt. Die organische Phase dreimal mit Wasser gewaschen, über Na₂SO₄ getrocknet, einrotiert, organische Phase abgetrennt, dreimal mit 200 mL Wasser gewaschen und anschließend zur Trockene eingeengt. Der Rückstand wird aus Heptan umkristallisiert. Ausbeute: 53 g (90 %)

### Schritt 2: 10-Biphenyl-4-yl-11,11-dimethyl-10,11-dihydro-5H-indolo[3,2-b]chinolin

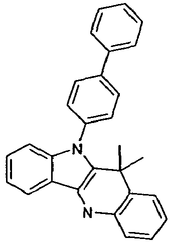

22.8 g (92 mmol) Wasserfreies Cer(III)chlorid werden in 700 mL trockenem THF vorgelegt. Zu dieser Lösung werden 35 g (84 mmol) 2-(1-Biphenyl-4-yl-1H-indol-3-ylamino)-benzoesäuremethylester portionsweise zudosiert und 1 h gerührt. Die Reaktionsmischung wird abgekühlt und bei 5 °C 117 mL (351 mmol) Methylmagnesiumchlorid-Lösung (3 mol/L in THF) innerhalb von 40 min zugetropft. Nach einer Stunde wird das Reaktionsgemisch vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 32.9 g (94 %)

28.9 g (250.9 mmol) Polyphosphorsäure und 16.5 mL Methansulfonsäure werden in 200 mL CH₂Cl₂ vorgelegt. Zu dieser Lösung werden 30 g (72 mmol) 2-[2-(1-Biphenyl-4-yl-1H-indol-3-ylamino)-phenyl]-propan-2-ol in CH₂Cl₂-Lösung (50 mL) innerhalb von 30 min zugetropft und 1 h bei 50 °C gerührt. Nach dieser Zeit wird das Reaktionsgemisch abgekühlt, vorsichtig 150 mL Ethanol zugegeben und dreimal mit Dichlormethan extrahiert. Die vereinigten organischen Phasen werden über Na₂SO₄ getrocknet und eingeengt. Ausbeute: 24,4 g 10-Biphenyl-4-yl-11,11-dimethyl-10,11-dihydro-5H-indolo[3,2-b]chinolin (85 %).

### Schritt 3: 5,10-Bis-biphenyl-4-yl-11,11-dimethyl-10,11-dihydro-5H-indolo3,2-bchinolin

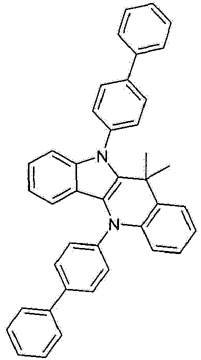

24 g (60 mmol) des Indolochinolin-Derivats, 15.4 g (66 mmol) Brombiphenyl und 16.6 g (191.7 mmol) NaOtBu werden in 500 mL Toluol suspendiert. Zu dieser Suspension werden 0.34 g (1.5 mmol) Pd(OAc)₂ und 3 mL einer 1M Tri-tert-butylphosphin Lösung gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99,9%.

### Beispiel 10 (nicht erfindungsmäßig): Verbindung 10

### Schritt 1: 1-Phenyl-3-(pyren-1-ylamino)-1H-indol-2-carbonsäure-methylester

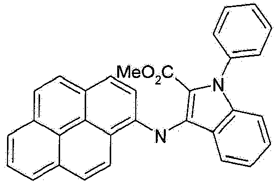

25 g (99 mmol) 1-Phenyl-1H-indol-2-carbonsäuremethylester werden in 400 mL Dichlormethan vorgelegt. Anschließend tropft man unter Lichtausschluss bei 0 °C eine Lösung aus 17.7 g (99 mmol) NBS in 100 ml Dichlormethan hinzu, lässt auf Raumtemperatur kommen und rührt 4 h weiter. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 32 g, (95 %)

30 g (91 mmol) 1-Phenyl-3-brom1-H-indol-2-carbonsäuremethylester, 10.7 g (91 mmol) Aminopyren und 59.2 g (181 mmol) Cs₂CO₃ werden in 800 mL Toluol suspendiert. Zu dieser Suspension werden 0.5 g (2.27 mmol) Palladiumacetat und 2.6 g (4.54 mmol) Xantphos gegeben. Die Reaktionsmischung wird 24 h unter Rückfluss erhitzt. Nach Erkalten wird die Mischung eingeengt und anschließend zwischen Essigester und Wasser verteilt. Die organische Phase dreimal mit Wasser gewaschen, über Na₂SO₄ getrocknet, einrotiert, organische Phase abgetrennt, dreimal mit 200 mL Wasser gewaschen und anschließend zur Trockene eingeengt. Der Rückstand wird aus Heptan umkristallisiert. Ausbeute: 36 g (85 %).

### Schritt 2:

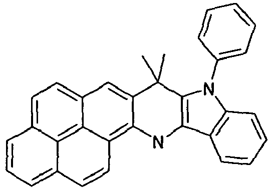

21.03 g (84.9 mmol) Wasserfreies Cer(III)-chlorid werden in 700 mL trockenem THF vorgelegt. Zu dieser Lösung werden 36 g (84 mmol) 1-Phenyl-3-(pyren-1-ylamino)-1H-indol-2-carbonsäuremethylester portionsweise zudosiert und 1 h gerührt. Die Reaktionsmischung wird abgekühlt und bei 5 °C 108 mL (324 mmol) Methylmagnesiumchlorid-Lösung (3 mol/L in THF) innerhalb von 40 min zugetropft. Nach 1 h wird das Reaktionsgemisch vorsichtig auf Eis gegossen und dreimal mit Dichlormethan extrahiert. Die vereinigten organischen Phasen werden über Na₂SO₄ getrocknet und eingeengt. Der Rückstand wird aus Toluol umkristallisiert. Ausbeute: 34.2 g (95 %)

26 g (225 mmol) Polyphosphorsäure und 14.8 mL (225 mmol) Methansulfonsäure werden in 200 mL CH₂Cl₂ vorgelegt. Zu dieser Lösung werden 30 g (54 mmol) 2-[1-Phenyl-3-(pyren-1-ylamino)-1H-indol-2-yl]-propan-2-ol in CH₂Cl₂-Lösung (100 mL) innerhalb von 30 min zugetropft und 1 h bei 50 °C gerührt. Nach dieser Zeit wird das Reaktionsgemisch abgekühlt, vorsichtig 150 mL Ethanol zugegeben und dreimal mit Dichlormethan extrahiert. Die vereinigten organischen Phasen werden über Na₂SO₄ getrocknet und eingeengt. Ausbeute: 27 g (76 %)

### Schritt 3:

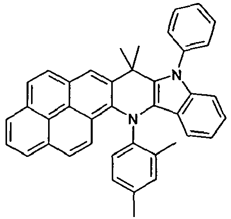

25 g (56 mmol) des Indoloquinolinderivats, 11.3 g (61 mmol) 1-Brom-2,4-dimethylphenyl und 175 g NaOtBu (178.3 mmol) werden in 500 mL Toluol suspendiert. Zu dieser Suspension werden 0.31 g (1.4 mmol) Pd(OAc)₂ und 2.8 mL eine 1M Tri-tert-butylphosphin Lösung gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert und aus Toluol umkristallisiert und abschließend im Hochvakuum sublimiert, Reinheit beträgt 99.9%.

### Beispiel 11: Herstellung der OLEDs

Die Herstellung von erfindungsgemäßen OLEDs erfolgt nach einem allgemeinen Verfahren gemäß WO 2004/058911, das auf die hier beschriebenen Gegebenheiten (Schichtdickenvariation, Materialien) angepasst wird.

In den folgenden Beispielen E1 bis E28 (siehe Tabellen 1 und 2) werden die Daten verschiedener OLEDs vorgestellt. Glasplättchen, die mit strukturiertem ITO (Indium-Zinn-Oxid) der Dicke 150 nm beschichtet sind werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / optionale Lochinjektionsschicht (HIL) / Lochtransportschicht (HTL) / optionale Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:6b:TER1 (65%:20%:15%) bedeutet hierbei, dass das Material ST1 in einem Volumenanteil von 65%, das Material 6b in einem Anteil von 20% und das Material TER1 in einem Anteil von 15% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet.

Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Je nach Substitutionsmuster lassen sich die erfindungsgemäßen Materialien in verschiedenen Schichten einsetzen. Dabei sind sehr gute Werte für Effizienz und Spannung erreichbar.

**Tabelle 1: Aufbau der OLEDs**

| Bsp. | HIL | HTL | IL | EBL | EML | HBL | ETL | EIL |
|---|---|---|---|---|---|---|---|---|
| | Dicke | Dicke | Dicke | Dicke | Dicke | Dicke | Dicke | Dicke |
| E1 | HATCN | SpA1 | --- | NPB | M2:D2 (90%:10%) | --- | 4b | LiQ |
| | 5nm | 110nm | | 20nm | 30nm | | 20nm | 3nm |
| E2 | --- | SpA1 | --- | NPB | ST1 :4d:TER2 | --- | Alq₃ | LiF |
| | | 20nm | | 20nm | (65%:20%:15%) 30nm | | 20nm | 1nm |
| E3 | --- | SpA1 | HATCN | BPA1 | 4e:TEG1 (90%:10%) | --- | ST2:LiQ (50%:50%) | --- |
| | | 70nm | 5nm | 90nm | 30nm | | 40nm | |
| E4 | --- | SpA1 | HATCN | BPA1 | 4e:TEG1 (90%:10%) | IC1 | ST2:LiQ (50%:50%) | --- |
| | | 70nm | 5nm | 90nm | 30nm | 10nm | 30nm | |
| E5 | --- | SpA1 | HATCN | BPA1 | ST1:41:TEG1 | IC1 | ST2:LiQ (50%:50%) | --- |
| | | 70nm | 5nm | 90nm | (30%:60%:10%) 30nm | 10nm | 30nm | |
| E6 | --- | SpA1 | HATCN | BPA1 | 4k:TEG1 (90%:10%) | --- | ST1:LiQ (50%:50%) | --- |
| | | 70nm | 5nm | 90nm | 30nm | | 40nm | |
| E7 | --- | SpA1 | --- | NPB | 4k:TER1 (85%:15%) | --- | Alq₃ | LiF |
| | | 20nm | | 20nm | 30nm | | 20nm | 1nm |
| E8 | --- | SpA1 | --- | NPB | 4m:TER1 (85%:15%) | --- | Alq₃ | LiF |
| | | 20nm | | 20nm | 30nm | | 20nm | 1nm |
| E9 | --- | SpA1 | --- | 4n | ST1:TER1 (85%:15%) | --- | Alq₃ | LiF |
| | | 20nm | | 20nm | 30nm | | 20nm | 1nm |
| E10 | --- | SpA1 | HATCN | BPA1 | ST1:TEG1 (90%:10%) | ST1 | 4o | LiQ |
| | | 70nm | 5nm | 20nm | 30nm | 10nm | 30 nm | 3nm |
| E11 | --- | SpA1 | BPA1 | 4q | ST1:TER1 (85%:15%) | --- | Alq₃ | LiF |
| | | 20nm | 10nm | 10nm | 30nm | | 20nm | 1nm |
| E12 | --- | SpA1 | --- | NPB | 5a:TER2 (85%:15%) | --- | Alq₃ | LiF |
| | | 20nm | | 20nm | 30nm | | 20nm | 1nm |
| E13 | --- | SpA1 | HATCN | BPA1 | IC1:6a:TEG1 | IC1 | ST2:LiQ (50%:50%) | --- |
| | | 70nm | 5nm | 90nm | (60%:30%:10%) 30nm | 10nm | 30nm | |
| E14 | --- | SpA1 | --- | NPB | ST1:6b:TER1 | ST1 | Alq₃ | LiF |
| | | 20nm | | 20nm | (65%:20%:15%) 30nm | 10nm | 20nm | 1nm |
| E15 | --- | SpA1 | HATCN | BPA1 | IC1:6c:TEG1 | IC1 | ST2:LiQ (50%:50%) | --- |
| | | 70nm | 5nm | 90 nm | (45%:45%:10%) 30nm | 10nm | 30nm | |
| E16 | --- | SpA1 | HATCN | 6d | ST1:TEG1 (90%:10%) | --- | ST1:LiQ (50%:50%) | --- |
| | | 70nm | 5nm | 20nm | 30nm | | 30 nm | |
| E17 | HATCN | SpA1 | --- | 6d | M2:D2 (90%:10%) | --- | Alq₃ | LiF |
| | 5nm | 110nm | | 20nm | 30nm | | 20nm | 1nm |
| E18 | --- | SpA1 | HATCN | 6d | M1:D1 (95%:5%) | --- | Alq₃ | LiF |
| | | 140nm | 5nm | 20nm | 20nm | | 30nm | 1nm |
| E19 | --- | SpA1 | HATCN | BPA1 | ST1:6e:TEG1 | --- | ST1:LiQ (50%:50%) | --- |
| | | 70nm | 5nm | 90nm | (50%:40%:10%) 30nm | | 40nm | |
| E20 | --- | 6f | --- | NPB | ST1:TER2 (85%:15%) | --- | Alq₃ | LiF |
| | | 20nm | | 20nm | 30nm | | 20nm | 1nm |
| E21 | --- | 6g | --- | NPB | ST1:TER2 (85%:15%) | --- | Alq₃ | LiF |
| | | 20nm | | 20nm | 30nm | | 20nm | 1nm |
| E22 | --- | SPA1 | --- | NPB | 6h:TER2 (85%:15%) | --- | Alq₃ | LiF |
| | | 20nm | | 20nm | 30nm | | 20nm | 1nm |
| E23 | --- | SpA1 | --- | NPB | ST1:6i:TER2 | ST2 | ST2:LiQ (50%:50%) | --- |
| | | 20nm | | 20nm | (70%:15%:15%) 30nm | 5nm | 25nm | |
| E24 | --- | SpA1 | --- | 6j | ST1:TER1 (85%:15%) | --- | Alq₃ | LiF |
| | | 20nm | | 20nm | 30nm | | 20nm | 1nm |
| E25 | --- | 9 | --- | NPB | ST1:TER2 (85%:15%) | --- | Alq₃ | LiF |
| | | 20nm | | 20nm | 30nm | | 20nm | 1nm |
| E26 | --- | SpA1 | HATCN | BPA1 | 8a:TEG1 (90%:10%) | IC1 | ST1:LiQ (50%:50%) | --- |
| | | 70nm | 5nm | 90nm | 30nm | 10nm | 30 nm | |
| E27 | --- | SpA1 | HATCN | BPA1 | 8a:IC2:TEG1 | IC1 | ST1:LiQ (50%:50%) | --- |
| | | 70nm | 5nm | 90nm | (20%:70%:10%) 30nm | 10nm | 30 nm | |
| E28 | HATCN | SpA1 | --- | NPB | M2:10 (97%:3%) | --- | ST2:LiQ (50%:50%) | --- |
| | 5nm | 140nm | | 20nm | 20nm | | 30nm | |

**Tabelle 2: Daten der OLEDs**

| Bsp. | U1000 (V) | SE1000 (cd/A) | LE1000 (Im/W) | EQE 1000 | CIE x/y |
|---|---|---|---|---|---|
| E1 | 5.4 | 17.7 | 10.3 | 5.2% | 0.29/0.61 |
| E2 | 5.6 | 11.3 | 6.4 | 10.5% | 0.66/0.33 |
| E3 | 3.8 | 50 | 41 | 13.8% | 0.36/0.61 |
| E4 | 3.9 | 52 | 41 | 14.3% | 0.3610.61 |
| E5 | 3.6 | 46 | 41 | 12.8% | 0.36/0.61 |
| E6 | 3.5 | 54 | 59 | 15.0% | 0.36/0.61 |
| E7 | 4.8 | 7.6 | 5.0 | 12.9% | 0.69/0.31 |
| E8 | 4.6 | 5.5 | 3.8 | 9.4% | 0.69/0.31 |
| E9 | 5.4 | 6.6 | 3.8 | 10.8% | 0.69/0.31 |
| E10 | 4.5 | 49 | 34 | 13.6% | 0.36/0.59 |
| E11 | 5.2 | 6.7 | 4.1 | 11.3% | 0.69/0.31 |
| E12 | 7.2 | 7.2 | 3.1 | 6.6% | 0.66/0.33 |
| E13 | 3.4 | 47 | 43 | 15.7% | 0.69/0.60 |
| E14 | 4.0 | 7.7 | 6.1 | 12.9% | 0.69/0.31 |
| E15 | 3.7 | 51 | 43 | 14.2% | 0.36/0.61 |
| E16 | 4.0 | 52 | 42 | 14.6% | 0.37/0.60 |
| E17 | 5.0 | 18.8 | 11.9 | 5.5% | 0.28/0.61 |
| E18 | 6.3 | 6.2 | 3.1 | 5.1% | 0.14/0.15 |
| E19 | 3.3 | 47 | 44 | 13.0% | 0.36/0.61 |
| E20 | 6.6 | 9.3 | 4.5 | 8.6% | 0.66/0.33 |
| E21 | 6.2 | 8.9 | 4.5 | 8.2% | 0.66/0.33 |
| E22 | 6.9 | 8.2 | 3.7 | 7.6% | 0.66/0.33 |
| E23 | 5.9 | 10.7 | 5.7 | 9.9% | 0.66/0.33 |
| E24 | 5.1 | 7.8 | 4.8 | 13.0% | 0.69/0.31 |
| E25 | 6.4 | 9.6 | 4.7 | 8.8% | 0.66/0.33 |
| E26 | 3.5 | 52 | 47 | 14.4% | 0.36/0.60 |
| E27 | 3.6 | 55 | 48 | 15.3% | 0.36/0.61 |
| E28 | 4.5 | 6.5 | 4.6 | 5.4% | 0.14/0.15 |

**Tabelle 3: Strukturformeln der Materialien für die OLEDs**

| | |
|---|---|
| 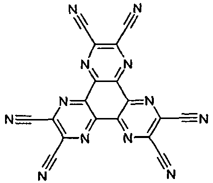 | 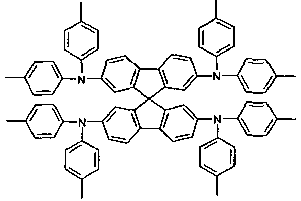 |
| HATCN | SpA1 |
| 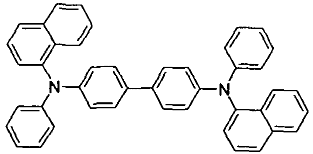 | 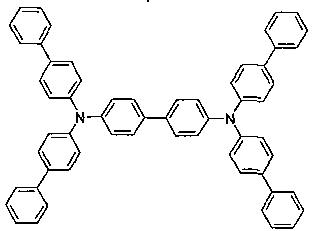 |
| NPB | BPA1 |
| 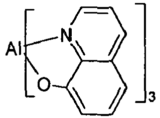 | 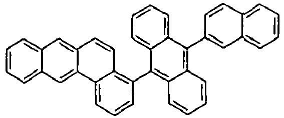 |
| Alq₃ | M1 |
| 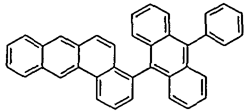 | 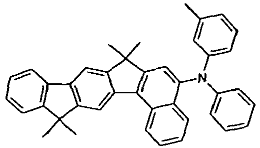 |
| M2 | D1 |
| 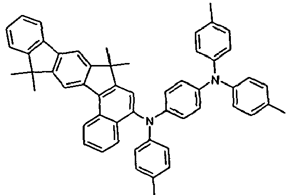 | 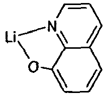 |
| D2 | LiQ |
| 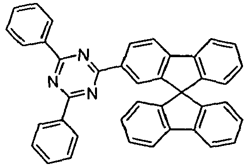 | 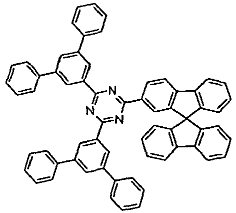 |
| ST1 | ST2 |
| 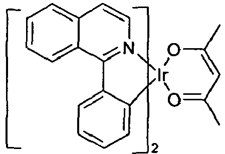 | 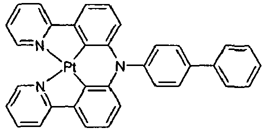 |
| TER1 | TER2 |
| 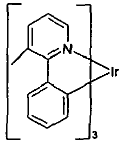 | 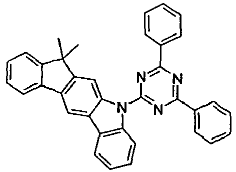 |
| TEG1 | IC1 |
| 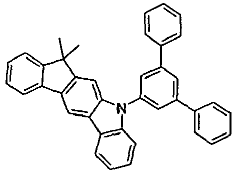 | |
| IC2 | |
| 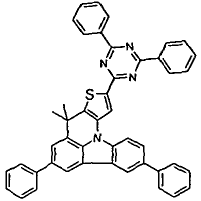 | 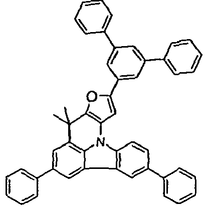 |
| 4b | 4d |
| 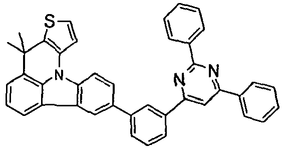 | 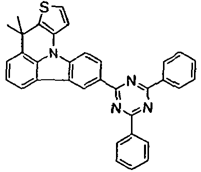 |
| 4e | 4k |
| 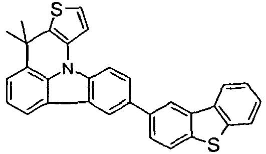 | 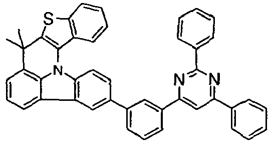 |
| 4l | 4m |
| 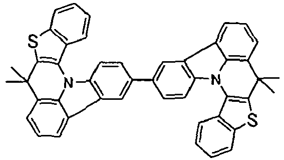 | 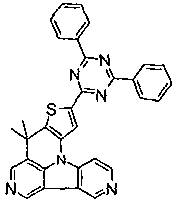 |
| 4n | 4o |
| 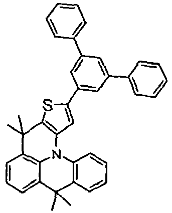 | 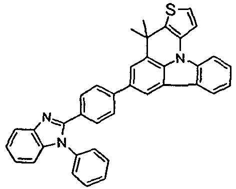 |
| 4q | 5a |
| 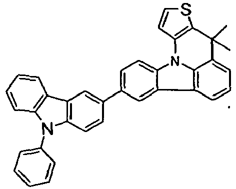 | 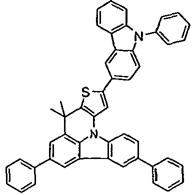 |
| 6a | 6b |
| 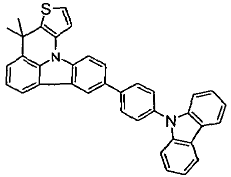 | 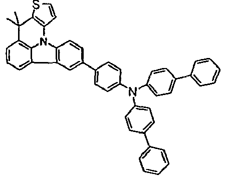 |
| 6c | 6d |
| 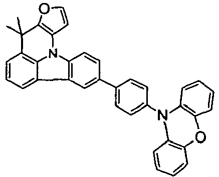 | 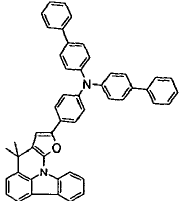 |
| 6e | 6f |
| 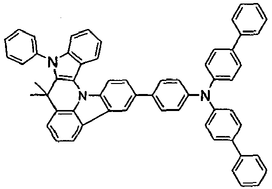 | 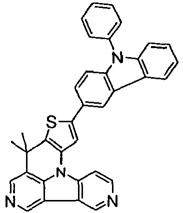 |
| 6g | 6h |
| 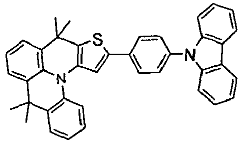 | 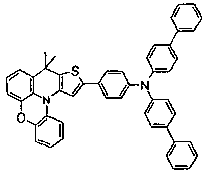 |
| 6i | 6j |
| 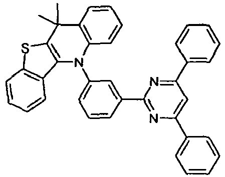 | 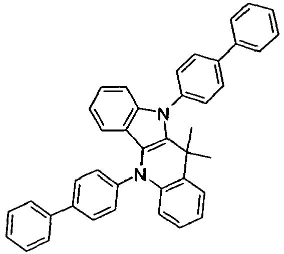 |
| 8a | 9 |
| 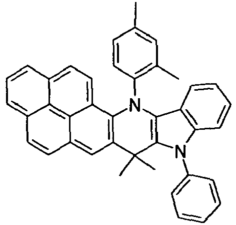 | |
| 10 | |

## Patentansprüche

1. Verbindung ausgewählt aus den Verbindungen der Formeln (5a) bis (11a), 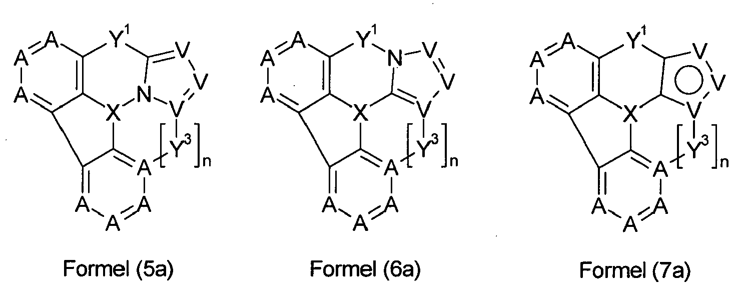 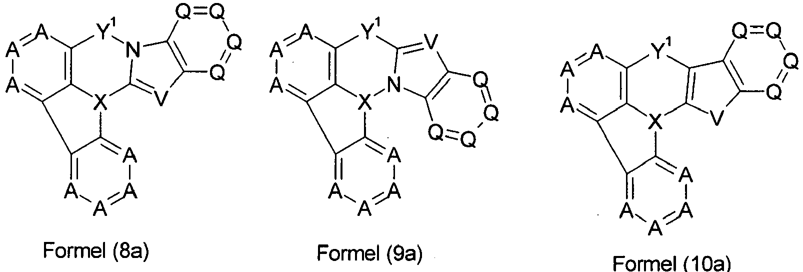 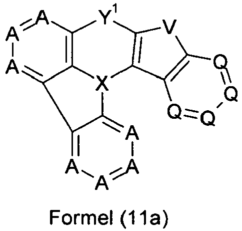 wobei für die verwendeten Symbole und Indizes gilt:
A ist bei jedem Auftreten gleich CR;
X ist N;
Y¹ ist C(R¹)₂;
V ist bei jedem Auftreten gleich oder verschieden CR, N, NR, S oder O, mit der Maßgabe, dass genau ein Symbol V für NR, S oder O steht;
Q ist bei jedem Auftreten gleich oder verschieden CR oder N;
R ist bei jedem Auftreten gleich oder verschieden ausgewählt aus der Gruppe bestehend aus H, D, F, CN, N(Ar)₂, C(=O)Ar, P(=O)(Ar)₂, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O oder S ersetzt sein können und wobei ein oder mehrere H-Atome durch D oder F ersetzt sein können, einem aromatischen oder heteroaromatischen Ringsystem mit 6 bis 30 aromatischen Ringatomen, das jeweils mit einem oder mehreren Resten R² substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R² substituiert sein kann;
R¹ ist ausgewählt aus der Gruppe bestehend aus H, Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R² substituiert sein können;
R² ist bei jedem Auftreten gleich oder verschieden ausgewählt aus der Gruppe bestehend aus H, D, F, Cl, Br, I, CN, NO₂, N(Ar)₂, N(R³)₂, C(=O)Ar, C(=O)R³, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder eine Aralkyl- oder Heteroaralkylgruppe mit 5 bis 60 aromatischen Ringatomen;
Ar ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar, welche an dasselbe N-Atom oder P-Atom binden, auch durch eine Einfachbindung oder eine Brücke, ausgewählt aus N(R³), C(R³)₂, O oder S, miteinander verbrückt sein;
R³ ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können;
n ist gleich 0.

2. Verbindung nach Anspruch 1, ausgewählt aus den Verbindungen der Formeln (5b) bis (11b), 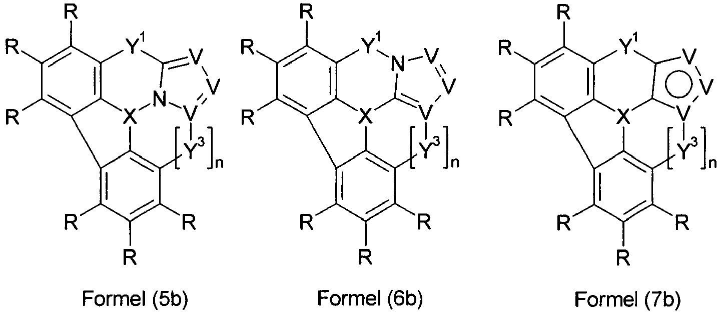 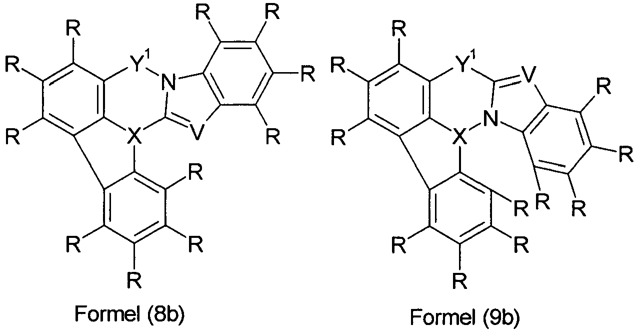 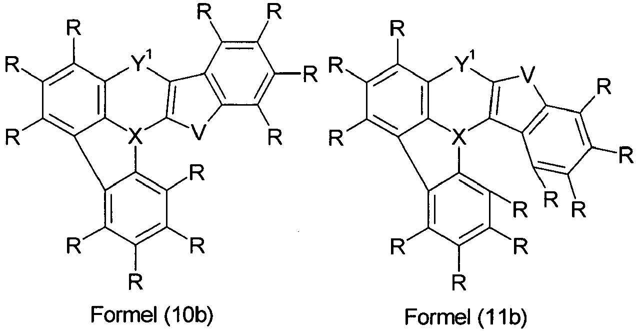 wobei die verwendeten Symbole und Indizes die in Anspruch 1 genannten Bedeutungen aufweisen.

3. Verbindung nach Anspruch 1 oder 2, **dadurch gekennzeichnet, dass** die Verbindung ausgewählt ist aus den Verbindungen gemäß den Formeln (5d) bis (11d), 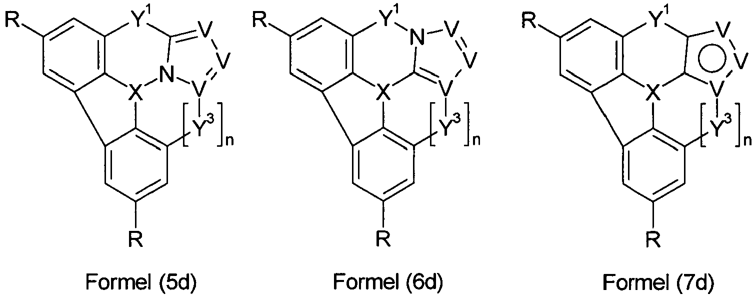 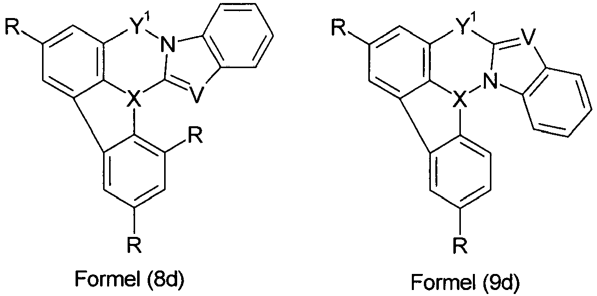 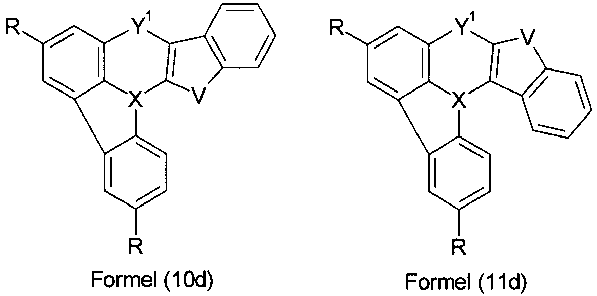 wobei die verwendeten Symbole und Indizes die in Anspruch 1 genannten Bedeutungen aufweisen.

4. Verbindung nach einem oder mehreren der Ansprüche 1 bis 3, **dadurch gekennzeichnet, dass** mindestens ein Rest R ausgewählt ist aus Strukturen der Formeln (12) bis (15), 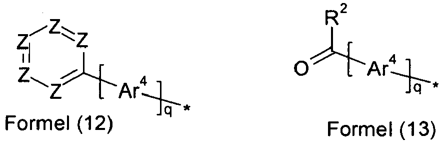 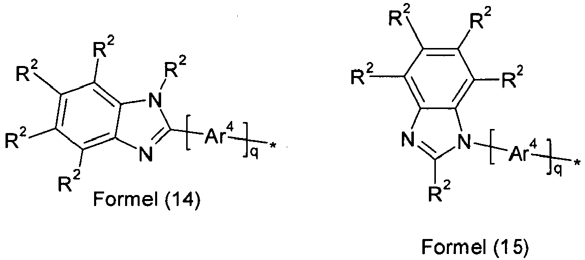 wobei R² die oben genannte Bedeutung hat, * die Position der Bindung der Gruppe gemäß Formel (12) bis (15) andeutet und weiterhin gilt:
Z ist bei jedem Auftreten gleich oder verschieden CR² oder N, mit der Maßgabe, dass eine Gruppe Z, zwei Gruppen Z oder drei Gruppen Z für N stehen;
Ar⁴ ist gleich oder verschieden bei jedem Auftreten eine bivalente Aryl- oder Heteroarylgruppe mit 5 bis 18 C-Atomen, welche durch einen oder mehrere Reste R² substituiert sein kann;
q ist bei jedem Auftreten gleich oder verschieden 0, 1, 2 oder 3;
und/oder dass mindestens ein Substituent R ausgewählt aus der Gruppe bestehend aus -NAr₂, Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R² substituiert sein können.

5. Verfahren zur Herstellung einer Verbindung nach einem oder mehreren der Ansprüche 1 bis 4, umfassend die Reaktionsschritte:
a) Synthese des Grundgerüsts, welches statt einer Gruppe R eine reaktive Abgangsgruppe trägt; und
b) Einführung der Gruppe R durch eine Kupplungsreaktion.

6. Mischung enthaltend mindestens eine Verbindung nach einem oder mehreren der Ansprüche 1 bis 4 und mindestens eine weitere Verbindung.

7. Formulierung enthaltend mindestens eine Verbindung nach einem oder mehreren der Ansprüche 1 bis 4 oder eine Mischung nach Anspruch 6 und ein oder mehrere Lösemittel.

8. Verwendung einer Verbindung nach einem oder mehreren der Ansprüche 1 bis 4 oder einer Mischung nach Anspruch 6 in einer elektronischen Vorrichtung.

9. Elektronische Vorrichtung enthaltend mindestens eine Verbindung nach einem oder mehreren der Ansprüche 1 bis 4 oder eine Mischung nach Anspruch 6.

10. Elektronische Vorrichtung nach Anspruch 9, **dadurch gekennzeichnet, dass** es sich um eine organische Elektrolumineszenzvorrichtung handelt und die Verbindung nach einem oder mehreren der Ansprüche 1 bis 4 oder die Mischung nach Anspruch 6 eingesetzt wird als Matrixmaterial für fluoreszierende oder phosphoreszierende Emitter in einer emittierenden Schicht und/oder als Lochblockiermaterial in einer Lochblockierschicht und/oder als Elektronentransportmaterial in einer Elektronentransportschicht und/oder als Elektronenblockier- bzw. Exzitonenblockiermaterial in einer elektronenblockierenden bzw. exzitonenblockierenden Schicht und/oder als Lochtransportmaterial in einer Lochtransportschicht oder in einer Lochinjektionsschicht.

## Claims

1. Compound selected from the compounds of the formulae (5a) to (11a), 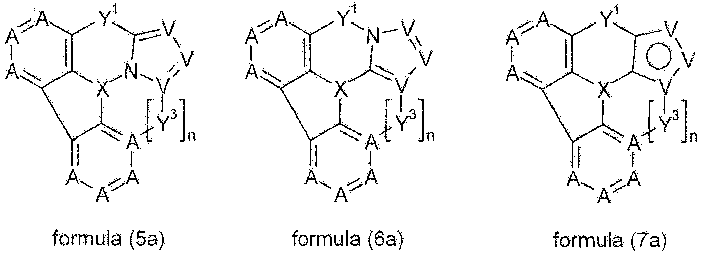 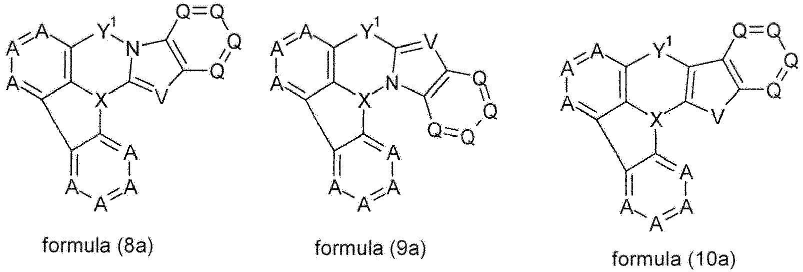 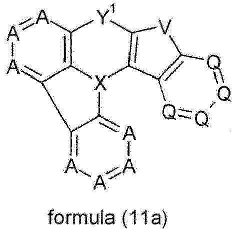 where the following applies to the symbols and indices used:
A is on each occurrence equal to CR;
X is N;
Y¹ is C(R¹)₂;
V is on each occurrence, identically or differently, CR, N, NR, S or O, with the proviso that precisely one symbol V stands for NR, S or O;
Q is on each occurrence, identically or differently, CR or N;
R is selected on each occurrence, identically or differently, from the group consisting of H, D, F, CN, N(Ar)₂, C(=O)Ar, P(=O)(Ar)₂, a straight-chain alkyl group having 1 to 10 C atoms or a branched or cyclic alkyl group having 3 to 10 C atoms or an alkenyl group having 2 to 10 C atoms, which may in each case be substituted by one or more radicals R², where one or more non-adjacent CH₂ groups may be replaced by O or S and where one or more H atoms may be replaced by D or F, an aromatic or heteroaromatic ring system having 6 to 30 aromatic ring atoms, which may in each case be substituted by one or more radicals R², where two or more adjacent substituents R may optionally form a monocyclic or polycyclic, aliphatic, aromatic or heteroaromatic ring system, which may be substituted by one or more radicals R², with one another;
R¹ is selected from the group consisting of H, alkyl groups having 1 to 10 C atoms or aromatic or heteroaromatic ring systems having 5 to 20 aromatic ring atoms, which may in each case be substituted by one or more radicals R²;
R² is selected on each occurrence, identically or differently, from the group consisting of H, D, F, Cl, Br, I, CN, NO₂, N(Ar)₂, N(R³)₂, C(=O)Ar, C(=O)R³, P(=O)(Ar)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R³, where one or more non-adjacent CH₂ groups may be replaced by R³C=CR³, C=C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or an aralkyl or heteroaralkyl group having 5 to 60 aromatic ring atoms;
Ar is on each occurrence, identically or differently, an aromatic or heteroaromatic ring system having 5-30 aromatic ring atoms, which may be substituted by one or more non-aromatic radicals R³; two radicals Ar which are bonded to the same N atom or P atom may also be bridged to one another by a single bond or a bridge selected from N(R³), C(R³)₂, O or S;
R³ is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN;
n is equal to 0.

2. Compound according to Claim 1, selected from the compounds of the formulae (5b) to (11b), 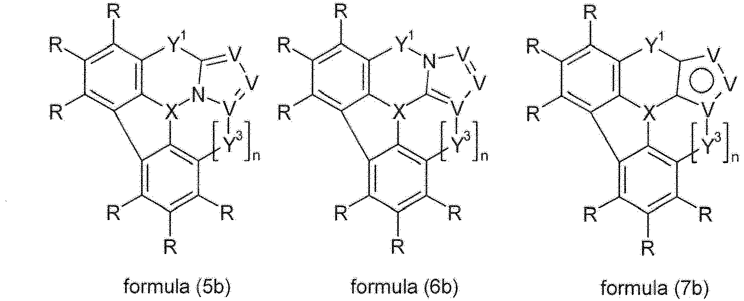 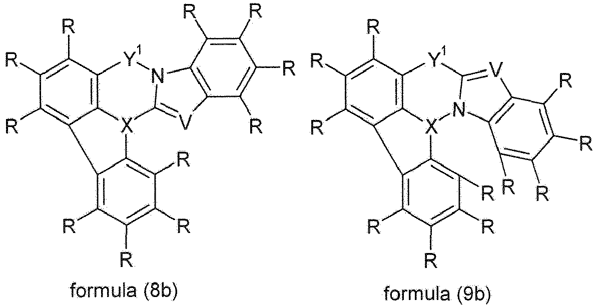 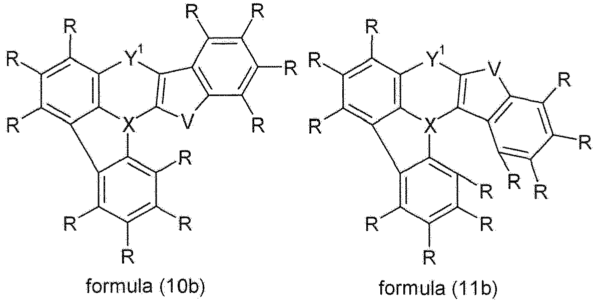 where the symbols and indices used have the meanings given in Claim 1.

3. Compound according to Claim 1 or 2, **characterised in that** the compound is selected from the compounds of the formulae (5d) to (11d), 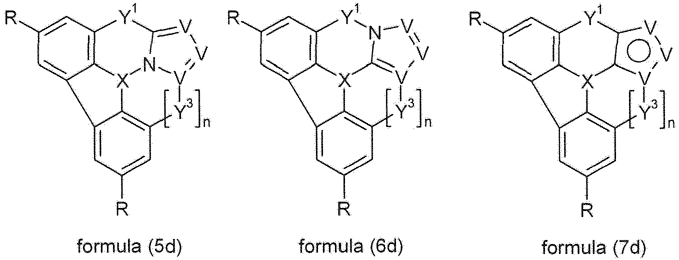 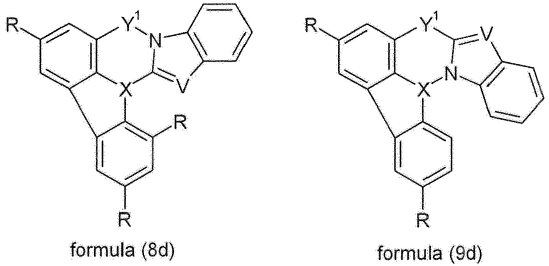 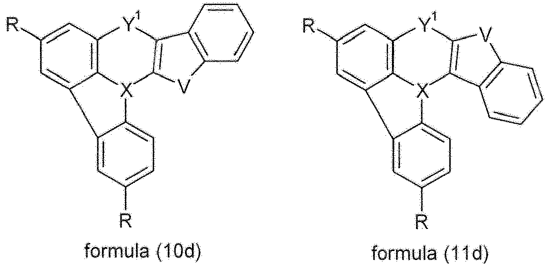 where the symbols and indices used have the meanings given in Claim 1.

4. Compound according to one or more of Claims 1 to 3, **characterised in that** at least one radical R is selected from structures of the formulae (12) to (15), 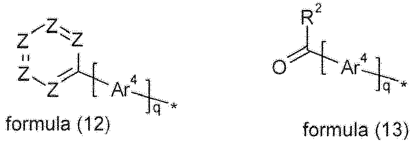 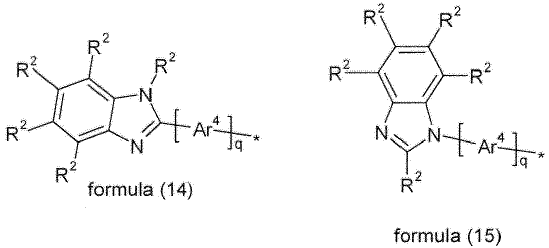 where R² has the above-mentioned meaning, * indicates the position of the bonding of the group of the formula (12) to (15) and furthermore:
Z is on each occurrence, identically or differently, CR² or N, with the proviso that one group Z, two groups Z or three groups Z stand for N;
Ar⁴ is, identically or differently on each occurrence, a divalent aryl or heteroaryl group having 5 to 18 C atoms, which may be substituted by one or more radicals R²;
q is on each occurrence, identically or differently, 0, 1, 2 or 3;
and/or **in that** at least one substituent R is selected from the group consisting of -NAr₂, triarylamine derivatives, carbazole derivatives, indenocarbazole derivatives, indolocarbazole derivatives, azacarbazole derivatives, indole derivatives, furan derivatives, benzofuran derivatives, dibenzofuran derivatives, thiophene derivatives, benzothiophene derivatives or dibenzothiophene derivatives, which may in each case be substituted by one or more radicals R².

5. Process for the preparation of a compound according to one or more of Claims 1 to 4, comprising the reaction steps:
a) synthesis of the skeleton which carries a reactive leaving group instead of a group R; and
b) introduction of the group R by a coupling reaction.

6. Mixture comprising at least one compound according to one or more of Claims 1 to 4 and at least one further compound.

7. Formulation comprising at least one compound according to one or more of Claims 1 to 4 or a mixture according to Claim 6 and one or more solvents.

8. Use of a compound according to one or more of Claims 1 to 4 or a mixture according to Claim 6 in an electronic device.

9. Electronic device containing at least one compound according to one or more of Claims 1 to 4 or a mixture according to Claim 6.

10. Electronic device according to Claim 9, **characterised in that** it is an organic electroluminescent device and the compound according to one or more of Claims 1 to 4 or the mixture according to Claim 6 is employed as matrix material for fluorescent or phosphorescent emitters in an emitting layer and/or as hole-blocking material in a hole-blocking layer and/or as electron-transport material in an electron-transport layer and/or as electron-blocking or exciton-blocking material in an electron-blocking or exciton-blocking layer and/or as hole-transport material in a hole-transport layer or in a hole-injection layer.

## Revendications

1. Composé sélectionné parmi les composés des formules (5a) à (11a) : 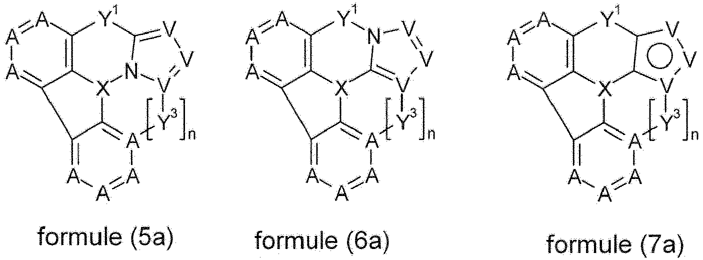 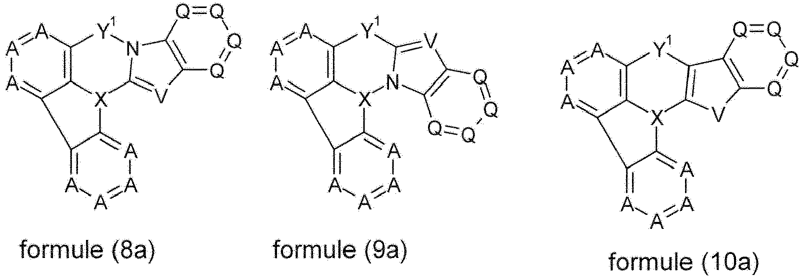 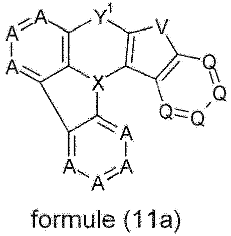 dans lesquelles ce qui suit s'applique aux symboles et aux indices qui sont utilisés :
A est pour chaque occurrence égal à CR ;
X est N ;
Y¹ est C(R¹)₂;
V est pour chaque occurrence, de manière identique ou différente, CR, N, NR, S ou O, étant entendu que précisément un symbole V représente NR, S ou O ;
Q est pour chaque occurrence, de manière identique ou différente, CR ou N ;
R est sélectionné pour chaque occurrence, de manière identique ou différente, parmi le groupe qui est constitué par H, D, F, CN, N(Ar)₂, C(=O)Ar, P(=O)(Ar)₂, un groupe alkyle en chaîne droite qui comporte de 1 à 10 atome(s) de C ou un groupe alkyle ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R², où un ou plusieurs groupe(s) CH₂ non adjacents peut/peuvent être remplacé(s) par O ou S et où un ou plusieurs atome(s) de H peut/peuvent être remplacé(s) par D ou F, un système de cycle aromatique ou hétéroaromatique qui comporte de 6 à 30 atomes de cycle aromatique, lequel peut dans chaque cas être substitué par un radical ou par plusieurs radicaux R², où deux substituants R adjacents ou plus peuvent en option former un système de cycle aliphatique, aromatique ou hétéroaromatique monocyclique ou polycyclique, lequel peut être substitué par un radical ou par plusieurs radicaux R², l'un avec l'autre ou les uns avec les autres ;
R¹ est sélectionné parmi le groupe qui est constitué par H, des groupes alkyle qui comportent de 1 à 10 atome(s) de C ou des systèmes de cycle aromatique ou hétéroaromatique qui comportent de 5 à 20 atomes de cycle aromatique, lesquels peuvent dans chaque cas être substitués par un radical ou par plusieurs radicaux R² ;
R² est sélectionné pour chaque occurrence, de manière identique ou différente, parmi le groupe qui est constitué par H, D, F, Cl, Br, I, CN, NO₂, N(Ar)₂, N(R³)₂, C(=O)Ar, C(=O)R³, P(=O)(Ar)₂, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, lequel peut dans chaque cas être substitué par un radical ou par plusieurs radicaux R³, où un ou plusieurs groupe(s) CH₂ non adjacents peut/peuvent être remplacé(s) par R³C=CR³, C≡C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atome(s) de H peut/ peuvent être remplacé(s) par D, F, Cl, Br, I, CN ou NO₂, un système de cycle aromatique ou hétéroaromatique qui comporte de 5 à 60 atomes de cycle aromatique, lequel peut dans chaque cas être substitué par un radical ou par plusieurs radicaux R³, ou un groupe aryloxy ou hétéroaryloxy qui comporte de 5 à 60 atomes de cycle aromatique, lequel peut être substitué par un radical ou par plusieurs radicaux R³, ou un groupe aralkyle ou hétéroaralkyle qui comporte de 5 à 60 atomes de cycle aromatique ;
Ar est pour chaque occurrence, de manière identique ou différente, un système de cycle aromatique ou hétéroaromatique qui comporte de 5 à 30 atomes de cycle aromatique, lequel peut être substitué par un radical ou plusieurs radicaux non aromatique(s) R³ ; deux radicaux Ar qui sont liés au même atome de N ou au même atome de P peuvent également être pontés l'un à l'autre au moyen d'une liaison simple ou d'un pont sélectionné parmi N(R³), C(R³)₂, O ou S ;
R³ est sélectionné parmi le groupe qui est constitué par H, D, F, CN, un radical hydrocarbone aliphatique qui comporte de 1 à 20 atome(s) de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I ou CN ;
n est égal à 0.

2. Composé selon la revendication 1, sélectionné parmi les composés des formules (5b) à (11b) : 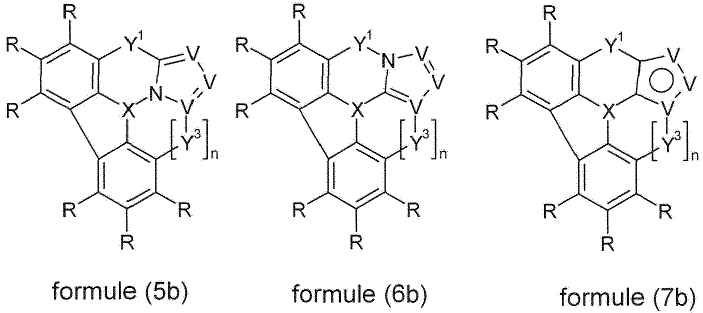 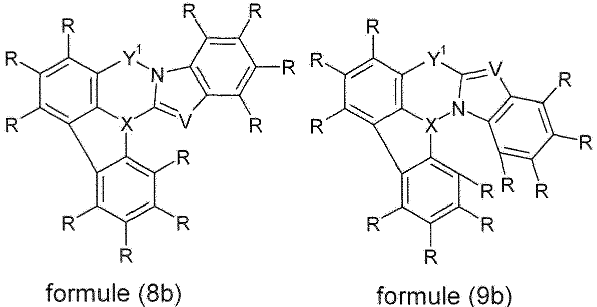 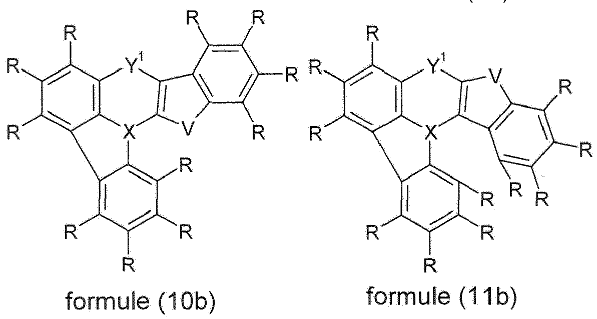 dans lesquelles les symboles et les indices qui sont utilisés présentent les significations qui ont été données selon la revendication 1.

3. Composé selon la revendication 1 ou 2, **caractérisé en ce que** le composé est sélectionné parmi les composés des formules (5d) à (11d) : 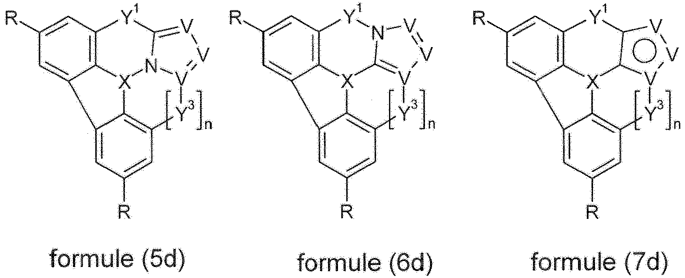 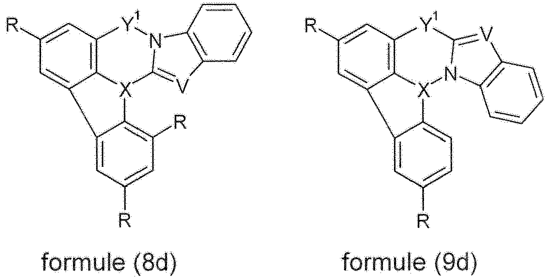 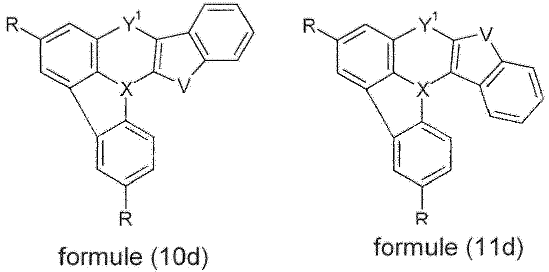 dans lesquelles les symboles et les indices qui sont utilisés présentent les significations qui ont été données selon la revendication 1.

4. Composé selon une ou plusieurs des revendications 1 à 3, **caractérisé en ce qu'**au moins un radical R est sélectionné parmi les structures des formules (12) à (15) : 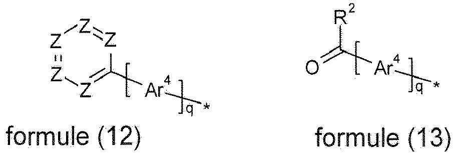 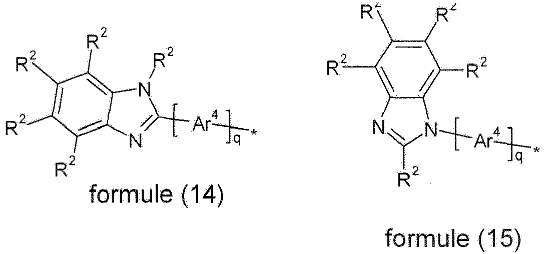 dans lesquelles R² présente la signification qui a été mentionnée ci-avant, * indique la position de la liaison du groupe des formules (12) à (15), et en outre :
Z est pour chaque occurrence, de manière identique ou différente, CR² ou N, étant entendu qu'un groupe Z, deux groupes Z ou trois groupes Z représente(nt) N ;
Ar⁴ est, de manière identique ou différente pour chaque occurrence, un groupe aryle ou hétéroaryle divalent qui comporte de 5 à 18 atomes de C, lequel peut être substitué par un radical ou par plusieurs radicaux R² ;
q est pour chaque occurrence, de manière identique ou différente, 0, 1, 2 ou 3 ;
et/ou **en ce qu'**au moins un substituant R est sélectionné parmi le groupe qui est constitué par -NAr₂, les dérivés de triarylamine, les dérivés de carbazole, les dérivées d'indénocarbazole, les dérivés d'indolocarbazole, les dérivés d'azacarbazole, les dérivés d'indole, les dérivés de furane, les dérivées de benzofurane, les dérivés de dibenzofurane, les dérivés de thiophène, les dérivés de benzothiophène ou les dérivés de dibenzothiophène, lesquels peuvent dans chaque cas être substitués par un radical ou par plusieurs radicaux R².

5. Procédé pour la préparation d'un composé selon une ou plusieurs des revendications 1 à 4, comprenant les étapes de réaction qui suivent :
a) la synthèse du squelette qui est porteur d'un groupe partant réactif en lieu et place d'un groupe R ; et
b) l'introduction du groupe R au moyen d'une réaction de couplage.

6. Mélange comprenant au moins un composé selon une ou plusieurs des revendications 1 à 4 et au moins un autre composé.

7. Formulation comprenant au moins un composé selon une ou plusieurs des revendications 1 à 4 ou un mélange selon la revendication 6 et un ou plusieurs solvant(s).

8. Utilisation d'un composé selon une ou plusieurs des revendications 1 à 4 ou d'un mélange selon la revendication 6 dans un dispositif électronique.

9. Dispositif électronique comportant au moins un composé selon une ou plusieurs des revendications 1 à 4 ou un mélanges selon la revendication 6.

10. Dispositif électronique selon la revendication 9, **caractérisé en ce qu'**il s'agit d'un dispositif électroluminescent organique, et le composé selon une ou plusieurs des revendications 1 à 4 ou le mélange selon la revendication 6 est utilisé en tant que matériau de matrice pour des émetteurs fluorescents ou phosphorescents dans une couche d'émission et/ou en tant que matériau de blocage de trous dans une couche de blocage de trous et/ou en tant que matériau de transport d'électrons dans une couche de transport d'électrons et/ou en tant que matériau de blocage d'électrons ou de blocage d'excitons dans une couche de blocage d'électrons ou dans une couche de blocage d'excitons et/ou en tant que matériau de transport de trous dans une couche de transport de trous ou dans une couche d'injection de trous.
